# EUROPEAN PATENT APPLICATION

(11) **EP 4 810 626 A1**
(43) Date of publication of application: **23.09.2026**
(21) Application number: 24911278.0
(22) Date of filing: 26.12.2024
(51) Int. Cl.: C12Q 1/6876, C40B 50/06, C12Q 1/6869

(54) **OLIGONUCLEOTIDE SET AND MULTIPLEXED AMPLICON LIBRARY CONSTRUCTION METHOD**

(30) Priority: 28.12.2023 CN 202311825749
(71) Applicant: Nanjing Vazyme Biotech Co., Ltd., Nanjing, Jiangsu 210046 (CN)
(72) Inventor: GUO, Jiayin, Nanjing, Jiangsu 210046 (CN); LI, Qi, Nanjing, Jiangsu 210046 (CN); WANG, Huiqi, Nanjing, Jiangsu 210046 (CN); YI, Wenyang, Nanjing, Jiangsu 210046 (CN)
(74) Representative: Buzzi, Notaro & Antonielli d'Oulx S.p.A.
(86) International application number: PCT/CN2024/142540
(87) International publication number: WO 2025/140356

(57) **Abstract**

Provided are an oligonucleotide set and a multiplexed amplicon library construction method, belonging to the technical field of biology. The provided method can, in a system, use a gene-specific multiplex amplification primer and a library amplification primer to, by means of a ligation or complementary extension mode, generate a ligation product or an extension product, and then, by means of the ligation product or the extension product being used as a long primer, complete construction of an amplicon library in one step. Compared with conventional multiplexed amplicon library construction methods, the provided method has the advantage of being able to decrease an uncovering operation step, avoiding the risk of cross contamination.

## Description

### TECHNICAL FIELD

The present disclosure relates to the technical field of biology, and in particular to an oligonucleotide set and a multiplexed amplicon library construction method.

### BACKGROUND

Current amplicon library construction is performed by means of two rounds of PCR amplification. In the first round of amplification, a gene-specific primer with a recognition sequence for the second-round primer (i.e., index sequence) is used to amplify a specific region. In the second round of amplification, a primer with a complementary sequence of the index sequence is used to amplify the product from the first round, thereby completing the library amplification. The problems associated with this most commonly used method are as follows: 1. After the first round of amplification, purification and recovery are required, followed by preparing the second-round system, performing the second round of amplification, and recovering the final product. In other words, two rounds of amplification and two rounds of recovery are involved, which increases the complexity of the operation. 2. Since the product from the first round of amplification carries universal sequences at both ends for recognition by second-round primers, when a large number of samples are amplified simultaneously, operations such as recovering the first-round product and preparing the reaction system are prone to cross-contamination via aerosols, thereby compromising the authenticity of the identification fragment signals between samples. 3. The numerous operational steps between the two rounds of amplification, along with the need to perform uncovering for sample loading, may cause cross-contamination between samples, thereby hindering the automation of targeted amplicon sequencing.

Chinese patent CN 104093890 B and U.S. patent US 10876108 B2 disclose an amplicon library construction method, which includes first performing library construction, and then amplifying specific fragments using a specific primer at one end and a universal primer at the other end. However, this library construction mode still requires multiple rounds to complete amplicon library construction, and is still associated with complex operational steps and aerosol cross-contamination. Especially in the application scenario of targeted next-generation sequencing (tNGS) for pathogens, aerosol-induced cross-contamination between samples severely hinders the automated application and large-scale promotion of targeted pathogen detection technologies.

Therefore, transforming the two-round amplification into a single-round amplification represents a technological development trend and helps solve the problem of cross-contamination between samples in targeted pathogen detection applications.

### SUMMARY

An object of the present disclosure is to provide an oligonucleotide pair and a multiplexed amplicon library construction method. By means of ligation or complementary extension, a gene-specific multiplex amplification primer and a library amplification primer in a multiplex amplification system are used to generate a ligation product or an extension product, and then, by means of the ligation product or the extension product being used as a long primer, the construction of an amplicon library in one step is completed. Compared with conventional multiplexed amplicon library construction methods, the method provided by the present disclosure has the advantage of being able to decrease an uncovering operation step, avoiding the risk of cross contamination.

A first aspect of the present disclosure involves an oligonucleotide set including a first oligonucleotide primer and a second oligonucleotide primer, where the first oligonucleotide primer includes, sequentially from the 5' end to the 3' end, a sequencing adapter sequence, an index sequence, and a sequencing primer sequence, and the second oligonucleotide primer includes, sequentially from the 5' end to the 3' end, a reverse complementary sequence of a gene-specific primer and a reverse complementary sequence of the sequencing primer.

In some embodiments, the first oligonucleotide primer and the second oligonucleotide primer are complementarily paired with each other through the sequencing primer sequence and the reverse complementary sequence of the sequencing primer.

In some embodiments, the "reverse complementary sequences" refer to nucleotide sequences that are associated according to the principle of base pairing. For example, the reverse complementary sequence of "5'-ATCG-3'" is "5'-CGAT-3'".

In some embodiments, the index sequence is a random sequence used to distinguish different samples to be tested. In some embodiments, the index sequence is at least 3 nucleotides, for example, 4-12 nucleotides, preferably 6-8 nucleotides, for example, 8 nucleotides.

In some embodiments, the gene-specific primer sequence is a specific primer sequence designed based on a target fragment of a DNA sample to be tested. For example, the gene-specific primer sequence includes at least 3 nucleotides, for example, 3-50 nucleotides, for example, 3, 5, 6, 7, 8, 9, 10, 11, 12, 13, 14, 15, 18, 20, 22, 25, 26, 28, 30, 32, 35, 38, 40, 42, 45, 48, and 50 nucleotides.

In some embodiments, one or more nucleotide sites of the reverse complementary sequence of the gene-specific primer contain an RNA base modification, where the modification is at least one of rA (riboadenosine), rU (ribouridine), rG (riboguanosine), and rC (ribocytidine). In some embodiments, the number of modifications is at least 1, for example, 1, 2, 3, 5, 6, 7, 8, 9, 10, 12, or 15.

In some embodiments, when multiple nucleotide sites of the reverse complementary sequence of the gene-specific primer contain RNA base modifications, the multiple nucleotide sites are continuous or discontinuous, and are each independently one of rA, rU, rG, and rC.

In some embodiments, one or more nucleotide sites in the second oligonucleotide primer contain an RNA base modification, where the modification is at least one of rA (riboadenosine), rU (ribouridine), rG (riboguanosine), and rC (ribocytidine). The number of modifications is at least 1, for example, 1, 2, 3, 5, 6, 7, 8, 9, 10, 12, 15, 16, 18, or 20.

In some embodiments, when multiple nucleotide sites in the second oligonucleotide primer contain RNA base modifications, the multiple nucleotide sites are continuous or discontinuous, and are each independently one of rA, rU, rG, and rC.

In some embodiments, the 3' end of the second oligonucleotide primer contains a blocking modification, which functions to prevent the addition of dNTPs to the 3' end of the second oligonucleotide primer for extension. In some embodiments, the blocking modification is a 3'-phosphorylation modification or a spacer arm modification; preferably, the spacer arm modification is a Spacer C3 modification, a Spacer C6 modification, a Spacer C9 modification, a dSpacer modification, or a PC-linker modification.

In some embodiments, the first oligonucleotide primer includes at least one pair of an upstream first oligonucleotide primer and a downstream first oligonucleotide primer.

In some embodiments, the upstream first oligonucleotide primer includes, sequentially from the 5' end to the 3' end, an upstream sequencing adapter sequence, an upstream index sequence, and an upstream sequencing primer sequence, and the downstream first oligonucleotide primer includes, sequentially from the 5' end to the 3' end, a downstream sequencing adapter sequence, a downstream index sequence, and a downstream sequencing primer sequence.

In some embodiments, the upstream first oligonucleotide primer does not include an (upstream) index sequence, and/or the downstream first oligonucleotide primer does not include a (downstream) index sequence.

In some embodiments, the sequencing adapter sequence is a sequencing substrate-associated sequence used in current or future sequencing platforms, including but not limited to the Ion Torrent platform, the Illumina platform, and the MGI platform (BGI).

In some embodiments, the sequencing adapter sequence includes an upstream sequencing adapter sequence and/or a downstream sequencing adapter sequence.

In some embodiments, the upstream sequencing adapter sequence is a P5 adapter sequence of the Illumina platform, and the downstream sequencing adapter sequence is a P7 adapter sequence of the Illumina platform; or *vice versa*.

In some embodiments, the upstream sequencing adapter sequence is a P1 adapter sequence of the ion torrent platform, and the downstream sequencing adapter sequence is an A adapter sequence of the ion torrent platform; or *vice versa*.

In some embodiments, the upstream sequencing adapter sequence is an upstream splint sequence for single-end tag library construction of the MGI platform, and the downstream sequencing adapter sequence is a downstream splint sequence for single-end tag library construction of the MGI platform; or *vice versa*. In some embodiments, the upstream sequencing adapter sequence is an upstream splint sequence for paired-end tag library construction of the MGI platform, and the downstream sequencing adapter sequence is a downstream splint sequence for paired-end tag library construction of the MGI platform; or *vice versa*.

In some embodiments, the upstream first oligonucleotide primer is an i5 primer of the Illumina platform, and the downstream first oligonucleotide primer is an i7 primer of the illumina platform; or *vice versa*.

In some embodiments, the second oligonucleotide primer includes at least one pair of an upstream second oligonucleotide primer and a downstream second oligonucleotide primer.

In some embodiments, the upstream second oligonucleotide primer includes, sequentially from the 5' end to the 3' end, a reverse complementary sequence of a gene-specific upstream primer and a reverse complementary sequence of the upstream sequencing primer.

In some embodiments, the downstream second oligonucleotide primer includes, sequentially from the 5' end to the 3' end, a reverse complementary sequence of a gene-specific downstream primer and a reverse complementary sequence of the downstream sequencing primer.

In some embodiments, the upstream first oligonucleotide primer and the upstream second oligonucleotide primer are complementarily paired with each other through the upstream sequencing primer sequence and the reverse complementary sequence of the upstream sequencing primer.

In some embodiments, the downstream first oligonucleotide primer and the downstream second oligonucleotide primer are complementarily paired with each other through the downstream sequencing primer sequence and the reverse complementary sequence of the downstream sequencing primer.

A second aspect of the present disclosure provides a composition including a DNA polymerase, an endoribonuclease, dNTPs, a buffer component, a metal salt, and a primer combination, where the primer combination includes the first oligonucleotide primer and the second oligonucleotide primer according to the first aspect, and in particular, at least one pair of an upstream first oligonucleotide primer and a downstream first oligonucleotide primer, and at least one pair of an upstream second oligonucleotide primer and a downstream second oligonucleotide primer.

In some embodiments, the first oligonucleotide primer and the second oligonucleotide primer are capable of complementary pairing with each other through the sequencing primer sequence and the reverse complementary sequence of the sequencing primer. In some embodiments, the first oligonucleotide primer and the second oligonucleotide primer each independently exist in the composition in a single-stranded form. In some embodiments, the first oligonucleotide primer and the second oligonucleotide primer exist in the composition in the form of a partial double-stranded structure formed by the sequencing primer sequence and the reverse complementary sequence of the sequencing primer.

In some embodiments, the first oligonucleotide primer includes at least one pair of the upstream first oligonucleotide primer and the downstream first oligonucleotide primer according to the first aspect, for example, at least 1, 5, 10, 20, 50, 100, 200, 300, and 500 pairs, including but not limited to any natural number between 1 and 1000.

In some embodiments, each pair of the upstream first oligonucleotide primer and the downstream first oligonucleotide primer differs from another pair in that the index sequences are not identical.

In some embodiments, the second oligonucleotide primer includes at least one pair of the upstream second oligonucleotide primer according to the first aspect and the downstream second oligonucleotide primer according to the first aspect, for example, at least 1, 5, 10, 20, 50, 100, 200, 300, and 500 pairs, including but not limited to any natural number between 1 and 1000.

In some embodiments, each pair of the upstream second oligonucleotide primer and the downstream second oligonucleotide primer differs from another pair in that the gene-specific primer sequences are not identical.

In some embodiments, the composition further includes a DNA sample.

In some embodiments, the DNA sample is gDNA or cDNA.

In some embodiments, the DNA polymerase includes at least one thermostable DNA polymerase.

In some embodiments, the DNA polymerase includes at least one thermostable DNA polymerase and one mesophilic DNA polymerase.

In some embodiments, the thermostable DNA polymerase is Taq DNA polymerase, Pfu DNA polymerase, Vent DNA polymerase, Deep Vent DNA polymerase, or KOD DNA polymerase.

In some embodiments, the thermostable polymerase is a hot-start polymerase, for example, an antibody-blocked polymerase or a ligand-blocked polymerase.

In some embodiments, the DNA polymerase includes at least one non-hot-start polymerase and one hot-start polymerase.

In some embodiments, the mesophilic DNA polymerase is Klenow DNA polymerase, Bst DNA polymerase, or Phi 29 DNA polymerase.

In some embodiments, the "mesophilic polymerase" described herein refers to a polymerase capable of exerting polymerase activity at a normal temperature, for example, below 45°C.

In some embodiments, the endoribonuclease recognizes an RNA/DNA hybrid strand and cleaves the RNA strand.

In some embodiments, the endoribonuclease is an endonuclease capable of recognizing a ribonucleotide site and cleaving a phosphodiester bond; preferably, the endoribonuclease is RNase H2.

In some embodiments, the endoribonuclease is a homologous protein of RNase H2.

In some embodiments, the buffer component is one or more of Tris, Tris-HCl, Tris base, or HEPES, preferably Tris.

In some embodiments, the metal salt is a Mg²⁺ salt, preferably MgCl₂.

In some embodiments, the metal salt is a Mg²⁺ salt, and one or more of a K⁺ salt, a Mn²⁺ salt, a Cs⁺ salt, a Na⁺ salt, and a Ca²⁺ salt.

In some embodiments, the composition further includes other components known in the art that can assist the DNA polymerase and endoribonuclease in functioning, for example, glycerol.

In some embodiments, the composition further includes a surfactant, for example, one or more of an anionic surfactant, a cationic surfactant, and a nonionic surfactant.

A third aspect of the present disclosure provides a multiplexed amplicon library construction method, the method including the steps of:
(1) preparing a reaction system including at least one pair of first oligonucleotide primers, at least one pair of second oligonucleotide primers, and a sample nucleic acid, where the first oligonucleotide primer includes, sequentially from the 5' end to the 3' end, a sequencing adapter sequence, an index sequence, and a sequencing primer sequence, the second oligonucleotide primer includes, sequentially from the 5' end to the 3' end, a reverse complementary sequence of a gene-specific primer and a reverse complementary sequence of the sequencing primer, and the second oligonucleotide primer includes a cleavable group;
(2) subjecting the 3' end of the first oligonucleotide primer and the 3' end of the second oligonucleotide primer to reverse complementary pairing, and extending the first oligonucleotide primer in the 5'-3' direction to obtain an extension product;
(3) cleaving the reverse complementary sequence of the gene-specific primer in the second oligonucleotide primer; and
(4) performing PCR amplification on the sample nucleic acid using the extension product described in step (2) as a primer.

In some embodiments, the index sequence is a random sequence used to distinguish different samples. In some embodiments, the index sequence is at least 3 nucleotides, for example, 4-12 nucleotides, preferably 6-8 nucleotides, for example, 8 nucleotides.

In some embodiments, the gene-specific primer sequence is a specific primer sequence designed based on a target fragment of a DNA sample to be tested. For example, the gene-specific primer sequence includes at least 3 nucleotides, for example, 3-50 nucleotides, for example, 3, 5, 6, 7, 8, 9, 10, 11, 12, 13, 14, 15, 18, 20, 22, 25, 26, 28, 30, 32, 35, 38, 40, 42, 45, 48, and 50 nucleotides.

In some embodiments, the 3' end of the second oligonucleotide primer contains a blocking modification, which functions to prevent the addition of dNTPs to the 3' end of the second oligonucleotide primer for extension. In some embodiments, the blocking modification is a 3'-phosphorylation modification or a spacer arm modification; preferably, the spacer arm modification is a Spacer C3 modification, a Spacer C6 modification, a Spacer C9 modification, a dSpacer modification, or a PC-linker modification.

In some embodiments, the at least one pair of first oligonucleotide primers includes at least one pair of an upstream first oligonucleotide primer and a downstream first oligonucleotide primer.

In some embodiments, the upstream first oligonucleotide primer includes, sequentially from the 5' end to the 3' end, an upstream sequencing adapter sequence, an upstream index sequence, and an upstream sequencing primer sequence, and the downstream first oligonucleotide primer includes, sequentially from the 5' end to the 3' end, a downstream sequencing adapter sequence, a downstream index sequence, and a downstream sequencing primer sequence.

In some embodiments, the upstream first oligonucleotide primer does not include an (upstream) index sequence, and/or the downstream first oligonucleotide primer does not include a (downstream) index sequence.

In some embodiments, the sequencing adapter sequence is a sequencing substrate-associated sequence used in current or future sequencing platforms, including but not limited to the Ion Torrent platform, the Illumina platform, and the MGI platform (BGI).

In some embodiments, the sequencing adapter sequence includes an upstream sequencing adapter sequence and a downstream sequencing adapter sequence.

In some embodiments, the upstream sequencing adapter sequence is a P5 adapter sequence of the Illumina platform, and the downstream sequencing adapter sequence is a P7 adapter sequence of the Illumina platform; or *vice versa*.

In some embodiments, the upstream sequencing adapter sequence is a P1 adapter sequence of the Ion Torrent platform, and the downstream sequencing adapter sequence is an A adapter sequence of the Ion Torrent platform; or *vice versa*.

In some embodiments, the upstream sequencing adapter sequence is an upstream splint sequence for single-end tag library construction of the MGI platform, and the downstream sequencing adapter sequence is a downstream splint sequence for single-end tag library construction of the MGI platform; or *vice versa*. In some embodiments, the upstream sequencing adapter sequence is an upstream splint sequence for paired-end tag library construction of the MGI platform, and the downstream sequencing adapter sequence is a downstream splint sequence for paired-end tag library construction of the MGI platform; or *vice versa*.

In some embodiments, the upstream first oligonucleotide primer is an i5 primer of the Illumina platform, and the downstream first oligonucleotide primer is an i7 primer of the Illumina platform; or *vice versa*.

In some embodiments, the at least one pair of second oligonucleotide primers includes at least one pair of an upstream second oligonucleotide primer and a downstream second oligonucleotide primer.

In some embodiments, the upstream second oligonucleotide primer includes, sequentially from the 5' end to the 3' end, a reverse complementary sequence of a gene-specific upstream primer and a reverse complementary sequence of the upstream sequencing primer.

In some embodiments, the downstream second oligonucleotide primer includes, sequentially from the 5' end to the 3' end, a reverse complementary sequence of a gene-specific downstream primer and a reverse complementary sequence of the downstream sequencing primer.

In some embodiments, the upstream first oligonucleotide primer and the upstream second oligonucleotide primer are complementarily paired with each other through the upstream sequencing primer sequence and the reverse complementary sequence of the upstream sequencing primer.

In some embodiments, the downstream first oligonucleotide primer and the downstream second oligonucleotide primer are complementarily paired with each other through the downstream sequencing primer sequence and the reverse complementary sequence of the upstream sequencing primer.

In some embodiments, the first oligonucleotide primer includes at least one pair of the upstream first oligonucleotide primer and the downstream first oligonucleotide primer according to the first aspect, for example, at least 1, 5, 10, 20, 50, 100, 200, 300, and 500 pairs, including but not limited to any natural number between 1 and 1000.

In some embodiments, each pair of the upstream first oligonucleotide primer and the downstream first oligonucleotide primer differs from another pair in that the index sequences are not identical.

In some embodiments, the second oligonucleotide primer includes at least one pair of the upstream second oligonucleotide primer and the downstream second oligonucleotide primer according to the first aspect, for example, at least 1, 5, 10, 20, 50, 100, 200, 300, and 500 pairs, including but not limited to any natural number between 1 and 1000.

In some embodiments, each pair of the upstream second oligonucleotide primer and the downstream second oligonucleotide primer differs from another pair in that the gene-specific primer sequences are not identical.

In some embodiments, the sample nucleic acid is DNA. For example, the DNA is gDNA or cDNA.

In some embodiments, the cleavable group refers to a nucleotide substrate that can be recognized and cleaved by an endonuclease or an exonuclease.

In some embodiments, the cleavable group is deoxyuridine triphosphate (dUTP) or deoxyinosine triphosphate (dITP). In some embodiments, when the cleavable group is dUTP, the reaction system further includes uracil DNA glycosylase (UDG) or single-strand selective monofunctional uracil-DNA glycosylase (SMUG); when the cleavable group is dITP, the reaction system further includes alkyladenine DNA glycosylase (AAG) or endonuclease V.

In some embodiments, the cleavable group is an RNA base, and the RNA base is at least one of rA (riboadenosine), rU (ribouridine), rG (riboguanosine), and rC (ribocytidine). In some embodiments, the number of RNA bases is at least 1, for example, 1, 2, 3, 5, 6, 7, 8, 9, 10, 12, or 15. In some embodiments, when the cleavable group is an RNA base, the reaction system further includes an endoribonuclease, where the endoribonuclease is an endonuclease capable of recognizing a ribonucleotide site and cleaving a phosphodiester bond; preferably, the endoribonuclease is RNase H2 or a homologous protein of RNase H2.

In some embodiments, the cleavable group is located in the reverse complementary sequence of the gene-specific primer and/or the reverse complementary sequence of the sequencing primer in the second oligonucleotide primer.

In some embodiments, step (3) includes cleaving the cleavable group on the reverse complementary sequence of the gene-specific primer in the second oligonucleotide primer under the action of an endonuclease or an exonuclease, for example, recognizing and cleaving a ribonucleotide site in the reverse complementary sequence of the gene-specific primer under the action of an endoribonuclease.

In some embodiments, the first oligonucleotide primer includes at least one pair of the upstream first oligonucleotide primer and the downstream first oligonucleotide primer, for example, at least 1, 5, 10, 20, 50, 100, 200, 300, and 500 pairs, including but not limited to any natural number between 1 and 1000.

In some embodiments, each pair of the upstream first oligonucleotide primer and the downstream first oligonucleotide primer differs from another pair in that the index sequences are not identical.

In some embodiments, the second oligonucleotide primer includes at least one pair of the upstream second oligonucleotide primer and the downstream second oligonucleotide primer, for example, at least 1, 5, 10, 20, 50, 100, 200, 300, and 500 pairs, including but not limited to any natural number between 1 and 1000.

In some embodiments, each pair of the upstream second oligonucleotide primer and the downstream second oligonucleotide primer differs from another pair in that the gene-specific primer sequences are not identical.

In some embodiments, the reaction system further includes a DNA polymerase.

In some embodiments, the DNA polymerase includes at least one thermostable DNA polymerase.

In some embodiments, the DNA polymerase includes at least one thermostable DNA polymerase and one mesophilic DNA polymerase.

In some embodiments, the thermostable polymerase is a hot-start polymerase, for example, an antibody-blocked polymerase or a ligand-blocked polymerase.

In some embodiments, the DNA polymerase includes at least one non-hot-start polymerase and one hot-start polymerase.

In some embodiments, the thermostable DNA polymerase is Taq DNA polymerase, Pfu DNA polymerase, Vent DNA polymerase, Deep Vent DNA polymerase, or KOD DNA polymerase.

In some embodiments, the mesophilic DNA polymerase is Klenow DNA polymerase, Bst DNA polymerase, or Phi 29 DNA polymerase.

In some embodiments, the reaction of step (2) is performed under the action of a non-hot-start polymerase; and the reaction of step (4) is performed under the action of a hot-start polymerase.

In some embodiments, the reaction of step (2) is performed under the action of a mesophilic polymerase; and the reaction of step (4) is performed under the action of a thermostable polymerase.

In some embodiments, the reaction temperature of step (2) is 20-50°C, preferably 25-40°C, for example, 25°C, 26°C, 27°C, 28°C, 29°C, 30°C, 31°C, 32°C, 33°C, 34°C, 35°C, 36°C, 37°C, 38°C, 39°C, or 40°C.

In some embodiments, the reaction time of step (2) is 5-40 minutes, preferably 10-30 minutes, for example, 10 minutes, 11 minutes, 12 minutes, 13 minutes, 14 minutes, 15 minutes, 16 minutes, 17 minutes, 18 minutes, 19 minutes, 20 minutes, 21 minutes, 22 minutes, 23 minutes, 24 minutes, 25 minutes, 26 minutes, 27 minutes, 28 minutes, 29 minutes, or 30 minutes.

In some embodiments, the reaction conditions of step (2) are incubation at 25-40°C for 10-30 minutes, incubation at 25-40°C for 10-25 minutes, incubation at 25-40°C for 10-20 minutes, or incubation at 30°C for 10-20 minutes, including but not limited to any combination of the reaction temperatures and the reaction times of step (2) described above.

In some embodiments, the reaction temperature of step (3) is 40-80°C, preferably 60-80°C, for example, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, or 80°C.

In some embodiments, the reaction time of step (3) is 5-30 minutes, preferably 5-20 minutes, for example, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, or 20 minutes.

In some embodiments, the reaction conditions of step (3) are incubation at 60-80°C for 10-20 minutes, incubation at 65-80°C for 10-20 minutes, incubation at 70-80°C for 10-20 minutes, incubation at 70-80°C for 10-15 minutes, incubation at 70-75°C for 10-15 minutes, incubation at 72°C for 10-15 minutes, or incubation at 72°C for 10 minutes, including but not limited to any combination of the reaction temperatures and the reaction times of step (3) described above.

In some embodiments, in the reaction process of step (2), the extension product generated by the complementary extension of the first oligonucleotide primer and the second oligonucleotide primer under the action of DNA polymerase is Index-Panel long primer, where the Index-Panel long primer includes, sequentially from the 5' end to the 3' end, a sequencing adapter sequence, an index sequence, a sequencing primer sequence, and a gene-specific primer sequence.

In some embodiments, the reaction system further includes a buffer component. In some embodiments, the buffer component is one or more of Tris, Tris-HCl, Tris base, or HEPES, preferably Tris.

In some embodiments, the reaction system further includes a metal salt. In some embodiments, the metal salt is a Mg²⁺ salt, preferably MgCl₂. In some embodiments, the metal salt is a Mg²⁺ salt, and one or more of a K⁺ salt, a Mn²⁺ salt, a Cs⁺ salt, a Na⁺ salt, and a Ca²⁺ salt.

In some embodiments, the reaction system further includes dNTP.

In some embodiments, the reaction system further includes other components known in the art that assist the DNA polymerase and endoribonuclease in functioning, for example, glycerol.

In some embodiments, the reaction system further includes a surfactant, for example, one or more of an anionic surfactant, a cationic surfactant, and a nonionic surfactant.

In some embodiments, the PCR amplification of step (4) includes one or more cycles (for example, 20, 21, 22, 23, 24, 25, 26, 27, 28, 29, 30, 31, 32, 33, 34, 35, 36, 37, 38, 39, and 40 cycles) of denaturation, annealing, and extension; preferably, the PCR amplification includes pre-denaturation and multiple cycles (for example, 20, 21, 22, 23, 24, 25, 26, 27, 28, 29, 30, 31, 32, 33, 34, 35, 36, 37, 38, 39, and 40 cycles) of denaturation, annealing, and extension.

In some embodiments, the denaturation is high-temperature denaturation, which is high-temperature denaturation at a temperature of at least 90°C, for example, 90°C, 91°C, 92°C, 93°C, 94°C, and 95°C.

In some embodiments, the denaturation simultaneously achieves: (1) denaturing the sample DNA from double-stranded to single-stranded; and (2) inactivating the RNase H2 protein.

In some embodiments, the PCR amplification is a single round of PCR amplification.

In some embodiments, the conditions for the PCR amplification may be any conventional PCR amplification conditions known to a person of ordinary skill in the art.

In some embodiments, the method further includes step (5): purifying the amplification product of the sample nucleic acid.

In some embodiments, the purification is performed by a magnetic bead-based method, a high-salt precipitation method, a spin column method, or a phenol-chloroform extraction method, preferably a magnetic bead-based method.

A fourth aspect of the present disclosure provides an oligonucleotide primer including, sequentially from the 5' end to the 3' end, a sequencing adapter sequence, an index sequence, a sequencing primer sequence, and a gene-specific primer sequence.

A fifth aspect of the present disclosure provides an oligonucleotide combination including a first oligonucleotide primer, a third oligonucleotide primer, and a bridge primer, where the first oligonucleotide primer includes, sequentially from the 5' end to the 3' end, a sequencing adapter sequence, an index sequence, and a sequencing primer sequence, the third oligonucleotide primer includes, sequentially from the 5' end to the 3' end, a universal sequence and a gene-specific primer sequence, and the bridge primer includes, sequentially from the 5' end to the 3' end, a reverse complementary sequence of the universal sequence and a reverse complementary sequence of the sequencing primer.

In some embodiments, the index sequence is a random sequence used to distinguish different samples. In some embodiments, the index sequence is at least 3 nucleotides, for example, 4-12 nucleotides, preferably 6-8 nucleotides, for example, 8 nucleotides.

In some embodiments, the gene-specific primer sequence is a specific primer sequence designed based on a target fragment of a DNA sample to be tested. For example, the gene-specific primer sequence includes at least 3 nucleotides, for example, 3-50 nucleotides, for example, 3, 5, 6, 7, 8, 9, 10, 11, 12, 13, 14, 15, 18, 20, 22, 25, 26, 28, 30, 32, 35, 38, 40, 42, 45, 48, and 50 nucleotides.

In some embodiments, the reverse complementary sequence of the universal sequence refers to a sequence that is partially or completely reverse complementary to the universal sequence, and the reverse complementary sequence of the sequencing primer refers to a sequence that is partially or completely reverse complementary to the sequencing primer.

In some embodiments, the 5' end of the third oligonucleotide primer contains or does not contain a phosphorylation modification.

In some embodiments, the 3' end of the bridge primer contains a blocking modification, which functions to prevent the addition of dNTPs to the 3' end of the bridge primer for extension. In some embodiments, the blocking modification is a 3'-phosphorylation modification or a spacer arm modification; preferably, the spacer arm modification is a Spacer C3 modification, a Spacer C6 modification, a Spacer C9 modification, a dSpacer modification, or a PC-linker modification.

In some embodiments, the universal sequence is a random sequence, and the universal sequence is not identical to, nor does it undergo complementary pairing with, the sequencing adapter sequence, the index sequence, and/or the sequencing primer sequence.

In some embodiments, the universal sequence is a fixed sequence, which functions to connect the first oligonucleotide primer and the third oligonucleotide primer by means of complementary pairing with the bridge primer; moreover, the universal sequence is not identical to, nor does it undergo complementary pairing with, the sequencing adapter sequence, index sequence, and/or sequencing primer sequence.

In some embodiments, the universal sequence is not identical to, nor does it undergo complementary pairing with, the target gene sequence.

In some embodiments, the base composition of the universal sequence is balanced.

In some embodiments, the length of the universal sequence is 5-200 bp, for example, 5 bp, 6 bp, 7 bp, 8 bp, 9 bp, 10 bp, 15 bp, 20 bp, 25 bp, 30 bp, 40 bp, 50 bp, 60 bp, 70 bp, 80 bp, 90 bp, 100 bp, 120 bp, 150 bp, 160 bp, 180 bp, and 200 bp.

In some embodiments, the first oligonucleotide primer includes at least one pair of an upstream first oligonucleotide primer and a downstream first oligonucleotide primer.

In some embodiments, the upstream first oligonucleotide primer includes, sequentially from the 5' end to the 3' end, an upstream sequencing adapter sequence, an upstream index sequence, and an upstream sequencing primer sequence, and the downstream first oligonucleotide primer includes, sequentially from the 5' end to the 3' end, a downstream sequencing adapter sequence, a downstream index sequence, and a downstream sequencing primer sequence.

In some embodiments, the upstream first oligonucleotide primer does not include an (upstream) index sequence, and/or the downstream first oligonucleotide primer does not include a (downstream) index sequence.

In some embodiments, the sequencing adapter sequence is a sequencing substrate-associated sequence used in current or future sequencing platforms, including but not limited to the ion torrent platform, the Illumina platform, and the MGI platform (BGI).

In some embodiments, the sequencing adapter sequence includes an upstream sequencing adapter sequence and a downstream sequencing adapter sequence.

In some embodiments, the upstream sequencing adapter sequence is a P5 adapter sequence of the Illumina platform, and the downstream sequencing adapter sequence is a P7 adapter sequence of the Illumina platform; or *vice versa*.

In some embodiments, the upstream sequencing adapter sequence is a P1 adapter sequence of the Ion Torrent platform, and the downstream sequencing adapter sequence is an A adapter sequence of the Ion Torrent platform; or *vice versa*.

In some embodiments, the upstream sequencing adapter sequence is an upstream splint sequence for single-end tag library construction of the MGI platform, and the downstream sequencing adapter sequence is a downstream splint sequence for single-end tag library construction of the MGI platform; or *vice versa*. In some embodiments, the upstream sequencing adapter sequence is an upstream splint sequence for paired-end tag library construction of the MGI platform, and the downstream sequencing adapter sequence is a downstream splint sequence for paired-end tag library construction of the MGI platform; or *vice versa*.

In some embodiments, the upstream first oligonucleotide primer is an i5 primer of the Illumina platform, and the downstream first oligonucleotide primer is an i7 primer of the Illumina platform; or *vice versa*.

In some embodiments, the third oligonucleotide primer includes at least one pair of an upstream third oligonucleotide primer and a downstream third oligonucleotide primer.

In some embodiments, the upstream third oligonucleotide primer includes, sequentially from the 5' end to the 3' end, an upstream universal sequence and a gene-specific upstream primer sequence.

In some embodiments, the downstream third oligonucleotide primer includes, sequentially from the 5' end to the 3' end, a downstream universal sequence and a gene-specific downstream primer sequence.

In some embodiments, the bridge primer includes at least one pair of an upstream bridge primer and a downstream bridge primer.

In some embodiments, the upstream bridge primer includes, sequentially from the 5' end to the 3' end, a reverse complementary sequence of the upstream universal sequence and a reverse complementary sequence of the upstream sequencing primer.

In some embodiments, the downstream bridge primer includes, sequentially from the 5' end to the 3' end, a reverse complementary sequence of the downstream universal sequence and a reverse complementary sequence of the downstream sequencing primer.

In some embodiments, the upstream sequencing primer is sequencing primer 1 of the TruSeq library structure, and the downstream sequencing primer is sequencing primer 2 of the TruSeq library structure; or *vice versa*.

In some embodiments, the upstream sequencing primer is sequencing primer 1 of the Nextera library structure, and the downstream sequencing primer is sequencing primer 2 of the Nextera library structure; or *vice versa*.

In some embodiments, the upstream sequencing primer is sequencing primer 1 of the TruSeq library structure, and the downstream sequencing primer is sequencing primer 2 of the Nextera library structure; or *vice versa*.

In some embodiments, the upstream sequencing primer is sequencing primer 1 of the Nextera library structure, and the downstream sequencing primer is sequencing primer 2 of the TruSeq library structure; or *vice versa*.

A sixth aspect of the present disclosure provides a composition including a DNA polymerase, T4 DNA ligase, ATP, dNTPs, a buffer component, a metal salt, and a primer combination, where the primer combination includes the first oligonucleotide primer, the third oligonucleotide primer, and the bridge primer according to the fifth aspect.

In some embodiments, the first oligonucleotide primer, the third oligonucleotide primer, and the bridge primer each independently exist in the composition in a single-stranded form. In some embodiments, the first oligonucleotide primer and the bridge primer are complementary through the sequencing primer sequence, and the third oligonucleotide primer and the bridge primer are complementary through the universal primer sequence, existing in the composition in the form of a three-primer double-stranded complementary structure.

In some embodiments, the first oligonucleotide primer includes at least one pair of the upstream first oligonucleotide primer and the downstream first oligonucleotide primer according to the fourth aspect, for example, at least 1, 5, 10, 20, 50, 100, 200, 300, and 500 pairs, including but not limited to any natural number between 1 and 1000.

In some embodiments, each pair of the upstream first oligonucleotide primer and the downstream first oligonucleotide primer differs from another pair in that the index sequences are not identical.

In some embodiments, the third oligonucleotide primer includes at least one pair of the upstream third oligonucleotide primer and the downstream third oligonucleotide primer according to the fifth aspect, for example, at least 1, 5, 10, 20, 50, 100, 200, 300, and 500 pairs, including but not limited to any natural number between 1 and 1000.

In some embodiments, each pair of the upstream third oligonucleotide primer and the downstream third oligonucleotide primer differs from another pair in that the gene-specific primer sequences are not identical.

In some embodiments, the bridge primer includes at least one pair, for example, at least 1 or 2 pairs, of the upstream bridge primer according to the fifth aspect and the downstream bridge primer according to the fourth aspect.

In some embodiments, each pair of the upstream bridge primer and the downstream bridge primer differs from another pair in that the sequencing primers are not identical.

In some embodiments, the composition further includes a DNA sample.

In some embodiments, the DNA sample is gDNA or cDNA.

In some embodiments, the 5' end of the third oligonucleotide primer contains or does not contain a phosphorylation modification.

In some embodiments, when the 5' end of the third oligonucleotide primer does not contain a phosphorylation modification, the composition further includes a substance capable of inducing a phosphorylation modification at the 5' end of the third oligonucleotide primer, for example, T4 polynucleotide kinase (T4 PNK).

In some embodiments, the mass ratio or molar ratio of the first oligonucleotide primer to the third oligonucleotide primer is N : 1, where N is a constant greater than or equal to 1 and less than or equal to 5, for example, 1, 2, 3, 4, and 5.

In some embodiments, the DNA polymerase is any thermostable DNA polymerase known in the art. In some embodiments, the thermostable DNA polymerase is one or more of Taq DNA polymerase, Pfu DNA polymerase, Vent DNA polymerase, Deep Vent DNA polymerase, and KOD DNA polymerase.

In some embodiments, the buffer component is one or more of Tris, Tris-HCl, Tris base, or HEPES, preferably Tris.

In some embodiments, the metal salt is a Mg²⁺ salt, preferably MgCl₂.

In some embodiments, the metal salt is a Mg²⁺ salt, and one or more of a K⁺ salt, a Mn²⁺ salt, a Cs⁺ salt, a Na⁺ salt, and a Ca²⁺ salt.

In some embodiments, the composition further includes other substances that can assist components in the composition, such as enzymes, in functioning, for example, glycerol.

In some embodiments, the composition further includes a surfactant, for example, one or more of an anionic surfactant, a cationic surfactant, and a nonionic surfactant.

A seventh aspect of the present disclosure provides a multiplexed amplicon library construction method, the method including the steps of:
(1) preparing a reaction system including at least one pair of first oligonucleotide primers, at least one pair of third oligonucleotide primers, at least one pair of bridge primers, and a sample nucleic acid, where the first oligonucleotide primer includes, sequentially from the 5' end to the 3' end, a sequencing adapter sequence, an index sequence, and a sequencing primer sequence, the third oligonucleotide primer including, sequentially from the 5' end to the 3' end, a universal sequence and a gene-specific primer sequence, and the bridge primer including, sequentially from the 5' end to the 3' end, a reverse complementary sequence of the universal sequence and a reverse complementary sequence of the sequencing primer;
(2) subjecting the 3' end of the first oligonucleotide primer and the 3' end of the bridge primer to reverse complementary pairing, subjecting the 5' end of the third oligonucleotide primer and the 5' end of the bridge primer to reverse complementary pairing, and connecting the first oligonucleotide primer and the third oligonucleotide primer to obtain a ligation product; and (3) performing PCR amplification on the sample nucleic acid using the ligation product described in step (2) as a primer.

In some embodiments, the index sequence is a random sequence used to distinguish different samples. In some embodiments, the index sequence is at least 3 nucleotides, for example, 4-12 nucleotides, preferably 6-8 nucleotides, for example, 8 nucleotides.

In some embodiments, the gene-specific primer sequence is a specific primer sequence designed based on a target fragment of a DNA sample to be tested. For example, the gene-specific primer sequence includes at least 3 nucleotides, for example, 3-50 nucleotides, for example, 3, 5, 6, 7, 8, 9, 10, 11, 12, 13, 14, 15, 18, 20, 22, 25, 26, 28, 30, 32, 35, 38, 40, 42, 45, 48, and 50 nucleotides.

In some embodiments, the reverse complementary sequence of the universal sequence refers to a sequence that is partially or completely reverse complementary to the universal sequence, and the reverse complementary sequence of the sequencing primer refers to a sequence that is partially or completely reverse complementary to the sequencing primer.

In some embodiments, the 5' end of the third oligonucleotide primer contains or does not contain a phosphorylation modification.

In some embodiments, the 3' end of the bridge primer contains a blocking modification, which functions to prevent the addition of dNTPs to the 3' end of the bridge primer for extension. In some embodiments, the blocking modification is a 3'-phosphorylation modification or a spacer arm modification; preferably, the spacer arm modification is a Spacer C3 modification, a Spacer C6 modification, a Spacer C9 modification, a dSpacer modification, or a PC-linker modification.

In some embodiments, the universal sequence is a random sequence, and the universal sequence is not identical to, nor does it undergo complementary pairing with, the sequencing adapter sequence, the index sequence, and/or the sequencing primer sequence.

In some embodiments, the universal sequence is a fixed sequence, which functions to connect the first oligonucleotide primer and the third oligonucleotide primer by means of complementary pairing with the bridge primer; moreover, the universal sequence is not identical to, nor does it undergo complementary pairing with, the sequencing adapter sequence, index sequence, and/or sequencing primer sequence.

In some embodiments, the universal sequence is not identical to, nor does it undergo complementary pairing with, the target gene sequence.

In some embodiments, the base composition of the universal sequence is balanced.

In some embodiments, the length of the universal sequence is 10-200 bp, for example, 10 bp, 15 bp, 20 bp, 25 bp, 30 bp, 40 bp, 50 bp, 60 bp, 70 bp, 80 bp, 90 bp, 100 bp, 120 bp, 150 bp, 160 bp, 180 bp, and 200 bp.

In some embodiments, the at least one pair of first oligonucleotide primers includes at least one pair of an upstream first oligonucleotide primer and a downstream first oligonucleotide primer.

In some embodiments, the upstream first oligonucleotide primer includes, sequentially from the 5' end to the 3' end, an upstream sequencing adapter sequence, an upstream index sequence, and an upstream sequencing primer sequence, and the downstream first oligonucleotide primer includes, sequentially from the 5' end to the 3' end, a downstream sequencing adapter sequence, a downstream index sequence, and a downstream sequencing primer sequence.

In some embodiments, the upstream first oligonucleotide primer does not include an (upstream) index sequence, and/or the downstream first oligonucleotide primer does not include a (downstream) index sequence.

In some embodiments, the sequencing adapter sequence is a sequencing substrate-associated sequence used in current or future sequencing platforms, including but not limited to the Ion Torrent platform, the Illumina platform, and the MGI platform (BGI).

In some embodiments, the at least one pair of sequencing adapter sequences includes at least one pair of an upstream sequencing adapter sequence and a downstream sequencing adapter sequence.

In some embodiments, the upstream sequencing adapter sequence is a P5 adapter sequence of the Illumina platform, and the downstream sequencing adapter sequence is a P7 adapter sequence of the Illumina platform; or *vice versa*.

In some embodiments, the upstream sequencing adapter sequence is a P1 adapter sequence of the Ion Torrent platform, and the downstream sequencing adapter sequence is an A adapter sequence of the Ion Torrent platform; or *vice versa*.

In some embodiments, the upstream sequencing adapter sequence is an upstream splint sequence for single-end tag library construction of the MGI platform, and the downstream sequencing adapter sequence is a downstream splint sequence for single-end tag library construction of the MGI platform; or *vice versa*. In some embodiments, the upstream sequencing adapter sequence is an upstream splint sequence for paired-end tag library construction of the MGI platform, and the downstream sequencing adapter sequence is a downstream splint sequence for paired-end tag library construction of the MGI platform; or *vice versa*.

In some embodiments, the upstream first oligonucleotide primer is an i5 primer of the Illumina platform, and the downstream first oligonucleotide primer is an i7 primer of the Illumina platform; or *vice versa*.

In some embodiments, the third oligonucleotide primer includes at least one pair of an upstream third oligonucleotide primer and a downstream third oligonucleotide primer.

In some embodiments, the upstream third oligonucleotide primer includes, sequentially from the 5' end to the 3' end, an upstream universal sequence and a gene-specific upstream primer sequence.

In some embodiments, the downstream third oligonucleotide primer includes, sequentially from the 5' end to the 3' end, a downstream universal sequence and a gene-specific downstream primer sequence.

In some embodiments, the at least one pair of bridge primers includes at least one pair of an upstream bridge primer and a downstream bridge primer.

In some embodiments, the upstream bridge primer includes, sequentially from the 5' end to the 3' end, a reverse complementary sequence of the upstream universal sequence and a reverse complementary sequence of the upstream sequencing primer.

In some embodiments, the downstream bridge primer includes, sequentially from the 5' end to the 3' end, a reverse complementary sequence of the downstream universal sequence and a reverse complementary sequence of the downstream sequencing primer.

In some embodiments, the upstream sequencing primer is sequencing primer 1 of the TruSeq library structure, and the downstream sequencing primer is sequencing primer 2 of the TruSeq library structure; or *vice versa*.

In some embodiments, the upstream sequencing primer is sequencing primer 1 of the Nextera library structure, and the downstream sequencing primer is sequencing primer 2 of the Nextera library structure; or *vice versa*.

In some embodiments, the upstream sequencing primer is sequencing primer 1 of the TruSeq library structure, and the downstream sequencing primer is sequencing primer 2 of the Nextera library structure; or *vice versa*.

In some embodiments, the upstream sequencing primer is sequencing primer 1 of the Nextera library structure, and the downstream sequencing primer is sequencing primer 2 of the TruSeq library structure; or *vice versa*.

In some embodiments, the at least one pair of first oligonucleotide primers includes at least one pair of the upstream first oligonucleotide primer and the downstream first oligonucleotide primer, for example, at least 1, 5, 10, 20, 50, 100, 200, 300, and 500 pairs, including but not limited to any natural number between 1 and 1000.

In some embodiments, each pair of the upstream first oligonucleotide primer and the downstream first oligonucleotide primer differs from another pair in that the index sequences are not identical.

In some embodiments, the at least one pair of third oligonucleotide primers includes at least one pair of the upstream third oligonucleotide primer and the downstream third oligonucleotide primer, for example, at least 1, 5, 10, 20, 50, 100, 200, 300, and 500 pairs, including but not limited to any natural number between 1 and 1000.

In some embodiments, each pair of the upstream third oligonucleotide primer and the downstream third oligonucleotide primer differs from another pair in that the gene-specific primer sequences are not identical.

In some embodiments, the at least one pair of bridge primers includes at least one pair, for example, at least 1 or 2 pairs, of the upstream bridge primer and the downstream bridge primer.

In some embodiments, each pair of the upstream bridge primer and the downstream bridge primer differs from another pair in that the sequencing primers are not identical.

In some embodiments, the reaction temperature of step (2) is 20-50°C, preferably 25-45°C, for example, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, or 45°C.

In some embodiments, the reaction time of step (2) is 1-60 minutes, preferably 5-40 minutes, for example, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes, 21 minutes, 22 minutes, 23 minutes, 24 minutes, 25 minutes, 26 minutes, 27 minutes, 28 minutes, 29 minutes, 30 minutes, 31 minutes, 32 minutes, 33 minutes, 34 minutes, 35 minutes, 36 minutes, 37 minutes, 38 minutes, 39 minutes, or 40 minutes.

In some embodiments, the reaction conditions of step (2) are incubation at 20-50°C for 1-60 minutes, incubation at 20-50°C for 5-40 minutes, incubation at 25-45°C for 5-40 minutes, incubation at 25-40°C for 5-40 minutes, incubation at 25-40°C for 5-35 minutes, incubation at 25-35°C for 5-35 minutes, incubation at 25-35°C for 5-30 minutes, or incubation at 37°C for 5-30 minutes, including but not limited to any combination of the incubation temperatures and incubation times described above.

In some embodiments, the reaction system further includes a DNA ligase, preferably T4 DNA ligase. In some embodiments, in the incubation process of step (2), the ligation product generated from the first oligonucleotide primer and the third oligonucleotide primer under the action of a bridge primer and T4 DNA ligase is an Index-T4-Panel long primer, where the Index-T4-Panel long primer includes, sequentially from the 5' end to the 3' end, a linker sequence, an index sequence, a sequencing primer sequence, a universal sequence, and a gene-specific primer sequence.

In some embodiments, the sample nucleic acid is DNA. For example, the DNA is gDNA or cDNA.

In some embodiments, the 5' end of the third oligonucleotide primer contains or does not contain a phosphorylation modification.

In some embodiments, when the 5' end of the third oligonucleotide primer does not contain a phosphorylation modification, the composition further includes a substance capable of inducing a phosphorylation modification at the 5' end of the third oligonucleotide primer, for example, T4 polynucleotide kinase (T4 PNK).

In some embodiments, the mass ratio or molar ratio of the first oligonucleotide primer to the third oligonucleotide primer is N : 1, where N is a constant greater than or equal to 1 and less than or equal to 5, for example, 1, 2, 3, 4, and 5.

In some embodiments, the reaction system further includes a DNA polymerase. In some embodiments, the DNA polymerase is any thermostable DNA polymerase known in the art. In some embodiments, the thermostable DNA polymerase is one or more of Taq DNA polymerase, Pfu DNA polymerase, Vent DNA polymerase, Deep Vent DNA polymerase, and KOD DNA polymerase.

In some embodiments, the reaction system further includes a buffer component. In some embodiments, the buffer component is one or more of Tris, Tris-HCl, Tris base, or HEPES, preferably Tris.

In some embodiments, the reaction system further includes a metal salt. In some embodiments, the metal salt is a Mg²⁺ salt, preferably MgCl₂. In some embodiments, the metal salt is a Mg²⁺ salt, and one or more of a K⁺ salt, a Mn²⁺ salt, a Cs⁺ salt, a Na⁺ salt, and a Ca²⁺ salt.

In some embodiments, the reaction system further includes dNTP.

In some embodiments, the reaction system further includes other components known in the art that assist the DNA polymerase and endoribonuclease in functioning, for example, glycerol.

In some embodiments, the reaction system further includes a surfactant, for example, one or more of an anionic surfactant, a cationic surfactant, and a nonionic surfactant.

In some embodiments, the PCR amplification includes one or more cycles (for example, 20, 21, 22, 23, 24, 25, 26, 27, 28, 29, 30, 31, 32, 33, 34, 35, 36, 37, 38, 39, and 40 cycles) of denaturation, annealing, and extension; preferably, the PCR amplification includes pre-denaturation and multiple cycles (for example, 20, 21, 22, 23, 24, 25, 26, 27, 28, 29, 30, 31, 32, 33, 34, 35, 36, 37, 38, 39, and 40 cycles) of denaturation, annealing, and extension.

In some embodiments, the PCR amplification is a single round of PCR amplification.

In some embodiments, the conditions for the PCR amplification may be any conventional PCR amplification conditions known to a person of ordinary skill in the art.

In some embodiments, the method further includes step (4): purifying the amplification product of the sample nucleic acid.

In some embodiments, the purification is performed by a magnetic bead-based method, a high-salt precipitation method, a spin column method, or a phenol-chloroform extraction method, preferably a magnetic bead-based method.

An eighth aspect of the present disclosure provides an oligonucleotide primer including, sequentially from the 5' end to the 3' end, an adapter sequence, an index sequence, a sequencing primer sequence, a universal sequence, and a gene-specific primer sequence.

A ninth aspect of the present disclosure provides the use of the method according to the third aspect or the seventh aspect in multiplexed amplicon library construction.

In the present disclosure, a sequence and a reverse complementary sequence thereof may be completely complementary or partially complementary, as long as subsequent extension and amplification can be achieved.

"Completely complementary" means that all nucleotide bases of the sequence are capable of pairing with all nucleotide bases of the corresponding reverse complementary sequence.

"Partially complementary" means that at least 30% (for example, 40%, 50%, 60%, 70%, 80%, 90%, 95%, or 99%), but not all, of the bases in a contiguous sequence of the sequence hybridize with the same number of bases in a contiguous sequence of the corresponding reverse complementary sequence.

The composition of the present disclosure is used in a multiplex amplification system.

### BRIEF DESCRIPTION OF THE DRAWINGS

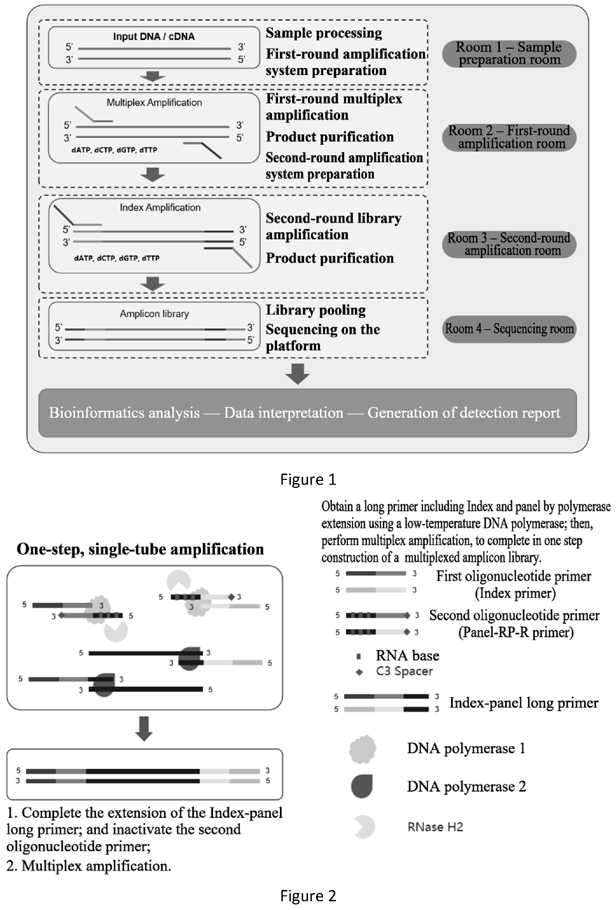

FIG. 1: Schematic diagram of the existing two-round multiplexed amplicon library construction;
FIG. 2: Schematic diagram of one-step multiplexed amplicon library construction based on a reverse Panel primer;
FIG. 3: Schematic diagram of one-step multiplexed amplicon library construction based on a bridge primer and T4 DNA ligase;
FIG. 4: Denaturing polyacrylamide gel electrophoresis images of primer extension in test groups 1-14 of Example 1;
FIG. 5: Denaturing polyacrylamide gel electrophoresis images of primer extension in test groups 1-20 of Example 2;
FIG. 6: Denaturing polyacrylamide gel electrophoresis images of primer extension in test groups 1-6 of Example 3;
FIG. 7: Gel electrophoresis images of amplification products in test groups 1-10 of Example 4;
FIG. 8: Denaturing polyacrylamide gel electrophoresis images after primer ligation in test groups 1-11 of Example 5;
FIG. 9: Gel electrophoresis images of amplification products in test groups 1-10 of Example 6;
FIG. 10: Final library structures of the 5TS-7NT group and the 5NT-7TS group;
FIG. 11: Gel electrophoresis images of amplification products in test groups 1-4 of Example 7;
FIG. 12: T4-Panel-16 primer sequences for constructing the 5NT-7TS library structure in Example 8;
FIG. 13: T4-Panel-16 primer sequences for constructing the 5TS-7NT library structure in Example 8;
FIG. 14: Panel-16 primer sequences containing only the specific sequences in Example 8;
FIG. 15: Gel electrophoresis images of one-step amplification products from the 16-plex Panel in test groups 1-13 of Example 8;
FIG. 16: Gel electrophoresis images of P5 and P7 primer amplification products in test groups 1-13 of Example 8;
FIG. 17: Reverse Panel primer sequences for library construction in Example 9;
FIG. 18: Panel primer sequences for library construction in Example 9;
FIG. 19: Gel electrophoresis images of amplification products in test groups 1-10 of Example 9;
FIG. 20: Peak profiles of target products from the one-step, single-tube method and the two-step method for library construction in Example 11;
FIG. 21: Number of reads detected by sequencing of libraries prepared by the one-step, single-tube method and the two-step method in Example 11;
FIG. 22: Number of dimers detected by sequencing of libraries prepared by the one-step, single-tube method and the two-step method in Example 11;
FIG. 23: Number of reads detected by sequencing of libraries prepared by the one-step, single-tube method and the two-step method in Example 10; and
FIG. 24: Number of dimers detected by sequencing of libraries prepared by the one-step, single-tube method and the two-step method in Example 10.

### DETAILED DESCRIPTION (EXAMPLES)

The technical solutions of the present disclosure will be further described below with reference to the accompanying drawings and through specific embodiments. However, the following examples are merely simple illustrations of the present disclosure and are not intended to represent or limit the scope of protection of the present disclosure, which shall be subject to the claims.

In the following examples, unless otherwise specified, all reagents and consumables used are purchased from conventional reagent manufacturers in the art; unless otherwise specified, all experimental methods and technical means used are conventional methods and means in the field.

### Example 1

### Generation of Index-Panel Long Primer by DNA Polymerase-Mediated Extension of Index Primer and Reverse Panel Primer

A conventional Vazyme #NA301 multiplex amplification system was used. Upstream and downstream Index primers (Vazyme #TD202) and the corresponding upstream and downstream reverse Panel primers (Panel-Rp) were added to the system. Further, Phanta DNA polymerase (Vazyme #P501) without antibody blocking was added. The amplification system and component volumes are shown in Table 1, and the types and lengths of primers in the system and in the product are shown in Table 2. Vazyme #NA301 includes a hot-start polymerase, and Vazyme #P501 is a non-hot-start polymerase. The primer sequences of Panel-Rp are respectively as follows (direction: 5'-3', the same below):
Panel-Rp-Forward (SEQ ID NO. 1):
   TGCTTATCAATGGATTTATATTTGTGACTGTCTCTTATACACATCTGACGCTGCCGACGA-C3 spacer
Panel-Rp-Reverse (SEQ ID NO. 2):
   CCCATTAGGATATTTCAAAGGTATATAAAGCTGTCTCTTATACACATCTCCGAGCCCACGAGAC-C3 spacer

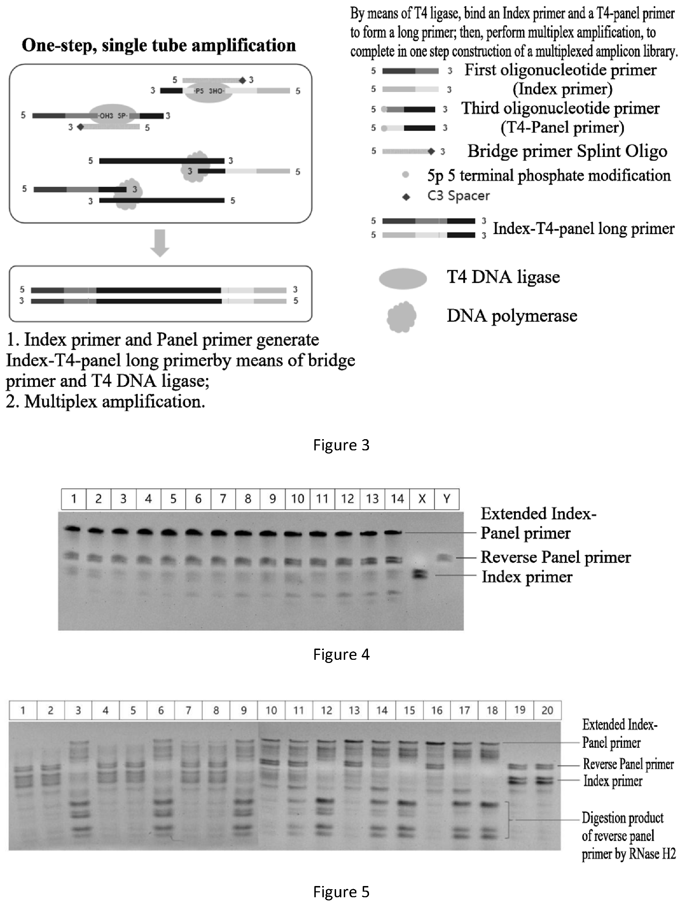

Test groups 1-14 with different reaction temperatures and reaction times were set up as shown in Table 3, and reactions were performed according to the corresponding temperatures and times. The product after reaction in each group was subjected to denaturing polyacrylamide gel electrophoresis. The results are shown in FIG. 4.

**Table 1**

| Component | Volume |
|---|---|
| Vazyme #NA301 | 15 µL |
| Index (i5 + i7, Vazyme #TD202) | 4 µL + 4 µL |
| Panel-Rp (10 µM) | 2 µL+ 2 µL |
| Phanta DNA polymerase (Vazyme #P501) | 1 U |
| ddH₂O | To 30 µL |

**Table 2**

| Primer type | Length | |
|---|---|---|
| | i5/Fwd | i7/Rev |
| Index | 51 nt | 47 nt |
| Panel-Rp | 60 nt | 64 nt |
| Index-Panel | 97 nt | 96 nt |

**Table 3**

| | Index + Panel-Rp | | | | | | | | | | | | | | Index | Panel -Rp |
|---|---|---|---|---|---|---|---|---|---|---|---|---|---|---|---|---|
| Reaction temperature | 25°C | | 37°C | | 45°C | | 50°C | | 55°C | | 60°C | | 65°C | | / | / |
| Reaction time (min) | 10 | 20 | 10 | 20 | 10 | 20 | 10 | 20 | 10 | 20 | 10 | 20 | 10 | 20 | / | / |
| Test group | 1 | 2 | 3 | 4 | 5 | 6 | 7 | 8 | 9 | 10 | 11 | 12 | 13 | 14 | X | Y |

As shown in FIG. 4, efficient extension of the Index-Panel long primer was achieved at temperatures ranging from 25°C to 65°C. This indicates that the reverse Panel primer can efficiently mediate the replication and production of the Index-Panel long primer.

In this example, the Index primer with the transposon-based library structure from Vazyme (Vazyme #TD202) was used for testing. In addition, this method is also applicable to conventional Index primers with the traditional Illumina library structure, such as Vazyme #N321/N322, and only requires designing a complementary sequence corresponding to the 3'-end sequence of the Index primer on the reverse Panel primer. Further, for the sequencing library structure of the MGI platform or other sequencing platforms known in the art, a similar approach as described above was used. Specifically, by designing a complementary sequence corresponding to the 3'-end sequence of the Index primer on the reverse Panel primer, one-step, single-tube multiplexed amplicon sequencing library construction was achieved.

### Example 2

### Removal of RNA Base-Modified Reverse Panel Primer by RNase H2

To further test the strategy for inactivating reverse Panel primer after the generation of the Index-Panel long primer through the reverse Panel primer, we selected upstream and downstream reverse Panel primers carrying RNA modifications (Panel-Rp-R) in combination with hot-start RNase H2 (IDT #11-03-02-03, an endoribonuclease that exhibits activity at elevated temperatures and can specifically recognize and cleave single RNA bases in double-stranded DNA) to inactivate the reverse Panel primer. To test the optimal reaction conditions for RNase H2, which must ensure that RNase H2 removes the reverse Panel primer only after DNA polymerase has preferentially completed the synthesis of the long Index-Panel primer, test groups 1-20 with different extension temperatures and reaction times were set up as shown in Table 5. The amplification system was prepared as shown in Table 5, and the reaction was performed according to the reaction program shown in Table 4. The sequences of Panel-Rp-R are respectively as follows:
Panel-Rp-R-Forward (SEQ ID NO. 3):
   TGCTTAT/rC/AATGGATTTA/rU/ATTTGTG/rA/CTGTCTCTTATACACATCTGACGCTGCCGACGA-C3 spacer
Panel-Rp-R-Reverse (SEQ ID NO. 4):

CCCATTA/rG/GATATTTCAAAGG/rU/ATATAAA/rG/CTGTCTCTTATACACATCTCCGAGCCCACGAGAC-
C3 spacer

The reaction products were subjected to denaturing polyacrylamide gel electrophoresis. The results are shown in FIG. 5.

**Table 4**

| | Index + Panel-Rp-R | | | | | | | | | | | | | | | | | | | |
|---|---|---|---|---|---|---|---|---|---|---|---|---|---|---|---|---|---|---|---|---|
| DNA Pol | Phanta DNA polymerase | | | | | | | | | | | | | | | | | | / | / |
| Extension temperatu re and time | 25°C 10 min | | | 30°C 10 min | | | 35°C 10 min | | | 40°C 10 min | | | 45°C 10 min | | | 50°C 10 min | | | 55°C 10 min | |
| RNase H2 | / | 3 U | | / | 3 U | | / | 3 U | | / | 3 U | | / | 3 U | | / | 3 U | | 3 U | / |
| Treatment time at 72°C | / | / | 10 min | / | / | 10 min | / | / | 10 min | / | / | 10 min | / | / | 10 min | / | / | 10 min | 10 min | 10 min |
| Test group | 1 | 2 | 3 | 4 | 5 | 6 | 7 | 8 | 9 | 10 | 11 | 12 | 1 3 | 1 4 | 15 | 1 6 | 1 7 | 18 | 19 | 20 |

**Table 5**

| Component | Volume |
|---|---|
| Vazyme #NA301 | 15 µL |
| Index i5 + i7 (Vazyme #TD202) | 4 µL + 4 µL |
| Panel-Rp-R (10 µM) | 2 µL+ 2 µL |
| Phanta DNA polymerase Vazyme #P501 | 1 U |
| RNase H2 | 3 U/- |
| ddH₂O | To 30 µL |

As described above, the optimal operating conditions for RNase H2 were tested in this example, which must ensure that RNase H2 remained inactive during the generation of the long primer by DNA polymerase. As can be seen from the results in FIG. 5, under the reaction conditions of test groups 1-9, no or only very weak digestion products of the reverse Panel primer were detected, regardless of whether RNase H2 was added, indicating that RNase H2 exhibited essentially no activity when the first-step reaction temperature was lower than 40°C. Therefore, 25-40°C can be selected as the temperature for the DNA polymerase extension reaction, which not only allows DNA polymerase to efficiently synthesize the Index-Panel long primer but also ensures that RNase H2 is in an inactive state. In contrast, when the reaction temperature was higher than 40°C, as shown in test groups 10-18 in FIG. 5, RNase H2 was able to exert RNA base cleavage activity. Consequently, the reverse Panel primer was cleaved by RNase H2 before the Index primer had completed extension into a long primer, causing the extension of the Index-Panel long primer to terminate at the RNA-modified base position. This ultimately led to the generation of incomplete Index-Panel long primers and subsequent nonspecific amplification. In summary, the complementary extension reaction of Panel-Rp-R primer and Index primer can be performed at 25-40°C, and the cleavage of Panel-Rp-R primer by RNase H2 can be performed at 40-72°C.

### Example 3

### Testing of Primer Extension and Removal Effect of Reverse Panel Primer Using a DNA Polymerase

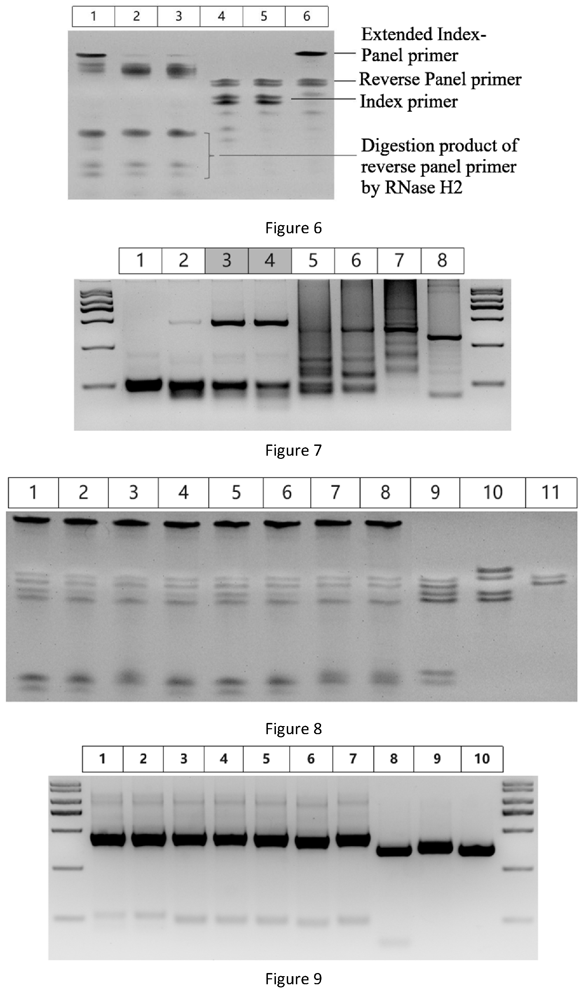

To test the generation of the Index-Panel long primer and the removal effect of the reverse Panel primer under conditions where the amplification system contained only one thermostable DNA polymerase, we selected the amplification system of Vazyme #PM202 in combination with a thermostable DNA polymerase (Vazyme #PM202) and hot-start RNase H2 (IDT #11-03-02-03) to set up test groups as shown in Table 6. The sequences of the Index and Panel-Rp-R primers used in the test were the same as those in Example 2. The prepared system is shown in Table 7. The reaction was performed according to the conditions described in the table, and the reaction products were subjected to denaturing polyacrylamide gel electrophoresis. The results are shown in FIG. 6.

**Table 6**

| Primer | Index + Panel-Rp-R | | | | | |
|---|---|---|---|---|---|---|
| Reaction condition | 50°C 10 min + 72°C 10 min | | | | | |
| DNA polymerase | 1 µL (Vazyme #PM202) | | | / | / | 1 µL (Vazyme #PM202) |
| RNase H2 | 3 U | 10 U | 30 U | 30 U | / | / |
| Test group | 1 | 2 | 3 | 4 | 5 | 6 |

**Table 7**

| Component | Volume |
|---|---|
| 2×PCR buffer (Vazyme #PM202) | 15 µL |
| DNA polymerase (Vazyme #PM202) | 1 µL/- |
| i5 + i7 (Vazyme #TD202) | 4 µL + 4 µL |
| Panel-Rp (10 µM) | 2 µL + 2 µL |
| RNase H2 | 3 U/10 U/30 U/- |
| dd H₂O | to 30 µL |

As shown in FIG. 6, efficient extension of the Index-Panel long primer was also achieved in an amplification system containing only one thermostable DNA polymerase.

### Example 4

### Testing of Primer Extension-Mediated One-step Amplification

Ten amplification experiments were further set up, including GSP primers (GSP-F/R), Panel primers, Index primer + Panel primer, Index primer + reverse Panel primer with RNA base modification (Panel-RP-R), and Index primer + reverse Panel primer without RNA base modification (Panel-RP). The specific settings, such as the addition amount of enzyme and the addition amounts of primers, for each group are shown in Table 8, the components and addition amounts of the amplification system are shown in Table 9, the amplification reaction program is shown in Table 10, and the descriptions and lengths of different primer sets are shown in Table 11. The sequences of some primers are as follows:
Panel-Rp-Forward (SEQ ID NO. 1):
   TGCTTATCAATGGATTTATATTTGTGACTGTCTCTTATACACATCTGACGCTGCCGACGA-C3 spacer
Panel-Rp-Reverse (SEQ ID NO. 2):
   CCCATTAGGATATTTCAAAGGTATATAAAGCTGTCTCTTATACACATCTCCGAGCCCACGAGAC-C3 spacer
Panel-Rp-R-Forward (SEQ ID NO. 3):
   TGCTTAT/rC/AATGGATTTA/rU/ATTTGTG/rA/CTGTCTCTTATACACATCTGACGCTGCCGACGA-C3 spacer
Panel-Rp-R-Reverse (SEQ ID NO. 4): 
CCCATTA/rG/GATATTTCAAAGG/rU/ATATAAA/rG/CTGTCTCTTATACACATCTCCGAGCCCACGAGAC-
C3 spacer
GSP-Forward (SEQ ID NO. 5): TCACAAATATAAATCCATTGATAAGCA
GSP-Reverse (SEQ ID NO. 6): CTTTATATACCTTTGAAATATCCTAATGGG
Panel-Forward (SEQ ID NO. 7):
   TCGTCGGCAGCGTCAGATGTGTATAAGAGACAGTCACAAATATAAATCCATTGATAAGCA
Panel-Reverse (SEQ ID NO. 8):
   GTCTCGTGGGCTCGGAGATGTGTATAAGAGACAGCTTTATATACCTTTGAAATATCCTAATGGG

The amplification products were subjected to agarose gel electrophoresis. The results are shown in FIG. 7.

**Table 8**

| | Index + Panel-Rp | Index + Panel-Rp-R | | | Index + Panel | | Panel | Gsp-F/R |
|---|---|---|---|---|---|---|---|---|
| DNA Pol | Phanta DNA polymerase | | | | | | | |
| RNase H2 | / | 3 U | | | / | | / | / |
| Index : Panel Primer ratio | 1: 1 | 1 : 2 | 2:1 | 3:1 | 1:1 | 2 : 1 | / | / |
| Treatment time | 72°C 10 min | | | | | | | |
| Test group | 1 | 2 | 3 | 4 | 5 | 6 | 7 | 8 |

**Table 9**

| Component | Volume |
|---|---|
| Vazyme #NA301 | 15 µL |
| Index, i5 + i7 (Vazyme #TD202) | 4 µL/- |
| Panel-Rp-R (10 µM) | 1 µL/2 µL/- |
| Panel-Rp (10 µM) | 1 µL/2 µL/- |
| Panel (10 µM) | 2 µL/- |
| GSP-F/R (10 µM) | 2 µL/- |
| Phanta DNA polymerase(Vazyme #P501) | 1 U |
| RNase H2 (IDT #11-03-02-03) | 3 U/- |
| 293T gDNA | 10 ng |
| ddH₂O | To 30 µL |

**Table 10**

| Temperature | Time | Number of cycles |
|---|---|---|
| 30°C | 10 min | 1 |
| 72°C | 10 min | 1 |
| 95°C | 3 min | 1 |
| 95°C | 30 s | 30 |
| 62°C | 30 s | |
| 72°C | 30 s | |
| 72°C | 3 min | 1 |
| 4°C | Hold | 1 |

**Table 11**

| Different primer sets | Primer meaning | Product length (bp) |
|---|---|---|
| Gsp-F/R | Gene-specific primer | 300 |
| Panel | Second oligonucleotide primer; including a sequencing primer sequence and a gene-specific primer sequence | 367 |
| Panel-Rp | Reverse complementary sequence of the second oligonucleotide primer | No amplification product |
| Index | First oligonucleotide primer, including a sequencing adapter sequence, an index sequence, and a sequencing primer sequence | No amplification product |
| Index-Panel | Long primer including a sequencing adapter sequence, an index sequence, a sequencing primer sequence and a gene-specific primer sequence | 436 |

As shown in FIG. 7, in test group 1, no amplification occurred without the addition of RNase H2 to remove the reverse Panel primer. In the test groups where RNase H2 was added, increasing the proportion of the Index primer resulted in an increased yield of the target product, as shown in test groups 3 and 4. In contrast, in test groups 5 and 6, where the Index and Panel primers were added directly to the system for amplification, a large number of nonspecific products were generated under the action of Phanta DNA polymerase.

### Example 5

### Testing of T4 DNA Ligase-Dependent Generation of Index-T4-Panel Long Primer

To achieve the generation of Index-T4-Panel long primer by connecting the Index and T4-Panel primers using a ligase, we designed a T4 DNA ligase-based method. Specifically, the Index primer and the T4-Panel primer were connected through a bridge primer (Splint Oligo, carrying an RNA base modification and a blocked 3' end, for example, a C3 spacer) using T4 DNA ligase to generate the Index-T4-Panel long primer. The T4-Panel primer includes a universal sequence and a gene-specific primer sequence, and the Panel primer includes a sequencing primer sequence and a gene-specific primer sequence. To explore the optimal ligation conditions for T4 DNA ligase, test groups 1-11 were set up according to Table 12. The components and amounts of the amplification system are shown in Table 13, the amplification program is shown in Table 14, and the descriptions and lengths of the primers are shown in Table 15. The sequences of some primers are as follows:
Panel-Forward (SEQ ID NO. 7):
   TCGTCGGCAGCGTCAGATGTGTATAAGAGACAGTCACAAATATAAATCCATTGATAAGCA
Panel-Reverse (SEQ ID NO. 8):
   GTCTCGTGGGCTCGGAGATGTGTATAAGAGACAGCTTTATATACCTTTGAAATATCCTAATGGG
Splint-Forward (SEQ ID NO. 9): TCTTGAGT/rG/AGACGCT/rG/CCGAC-C3 spacer
Splint-Reverse (SEQ ID NO. 10): GCTGTTG/rA/TGCCGA/rG/CCCAC-C3 spacer
T4-Panel-Forward (SEQ ID NO. 11): 5p-
   TCACTCAAGAAGATGTGTATAAGAGACAGTCACAAATATAAATCCATTGATAAGCA
T4-Panel-Reverse (SEQ ID NO. 12): 5p-
   CATCAACAGCAGATGTGTATAAGAGACAGCTTTATATACCTTTGAAATATCCTAATGGG

The amplification products were subjected to denaturing polyacrylamide gel electrophoresis. The results are shown in FIG. 8.

**Table 12**

| | Index + Splint + T4-Panel | | | | | | | | | Index + Panel | T4-Panel |
|---|---|---|---|---|---|---|---|---|---|---|---|
| T4 ligation conditions | 25° C 30 min | 37° C 30 min | 45° C 30 min | 37° C 30 min | 25°C 30 min | 37°C 30 min | 45°C 30 min | 45° C 30 min | 37°C 30 min | 37°C 30 min | 37°C 30 min |
| T4 DNA Ligase + ATP | 5 µg + 0.25 mM | | | 5 µg + 0.5 mM | 10 µg + 0.5 mM | | | 10 µg + 1 mM | / | / | / |
| Test group | 1 | 2 | 3 | 4 | 5 | 6 | 7 | 8 | 9 | 10 | 11 |

**Table 13**

| Component | Volume |
|---|---|
| Vzayme #NA301 | 15 µL |
| Index, (i5 + i7) (Vazyme #TD202) | 4 µL + 4 µL |
| Splint Oligo (10 µM) | 2 µL+ 2 µL |
| T4-Panel (10 µM) | 2 µL+ 2 µL |
| Panel (10 µM) | 2 µL/- |
| T4 Ligase (Vazyme #C301) | 5/10 µg |
| ATP | 0.25/0.5/1 mM |
| ddH₂O | To 30 µL |

**Table 14**

| Temperature | Time | Number of cycles |
|---|---|---|
| 25/37/45°C | 30 min | 1 |
| 4°C | - | 1 |

**Table 15**

| Primer type | Length | |
|---|---|---|
| | i5/Fwd | i7/Rev |
| Index | 51 nt | 47 nt |
| Panel | 60 nt | 64 nt |
| T4-Panel | 56 nt | 59 nt |
| Index-T4-Panel | 107 nt | 106 nt |

As shown in FIG. 8, different amounts of ligase and different ligation conditions were tested in this example. The denaturing page gel analysis results showed that T4 DNA ligase was able to efficiently connect the Index primer and the T4-Panel primer under all of the conditions tested.

### Example 6

### Testing of T4 DNA Ligase-Mediated One-Step Amplification

To test the amplification efficiency of the Index-T4-Panel long primer generated using our T4 DNA ligase-mediated strategy, ten amplification experiments were further set up, including Index primer + Splint Oligo (bridge primer) + T4-Panel primer, T4-Panel primer, and Panel primer + Index primer. The specific settings, such as the addition amount of enzyme and the addition amounts of primers, for each group are shown in Table 16, the components and addition amounts of the amplification system are shown in Table 17, the amplification reaction program is shown in Table 18, and the descriptions and lengths of the different primer sets are shown in Table 19. The primer sequences were the same as those in Example 5.

The amplification products were subjected to agarose gel electrophoresis. The results are shown in FIG. 9.

**Table 16**

| | Index + Splint Oligo + T4-Panel Primer | | | | | | | | Index + Panel | T4-Panel |
|---|---|---|---|---|---|---|---|---|---|---|
| T4 ligation conditions | 37°C 30 min | | | 37° C 5 min | 37°C 15 min | 37°C 20 min | 37°C 25 min | 37°C 30 min | 37°C 30 min | 37°C 30 min |
| RNase H2 Digestion conditions | 50°C 10 min | 60°C 10 min | / | / | / | / | / | / | / | / |
| T4 DNA Ligase + ATP | 5 µg + 0.25 mM | | | | | | | / | / | / |
| Test group | 1 | 2 | 3 | 4 | 5 | 6 | 7 | 8 | 9 | 10 |

**Table 17**

| Component | Addition amount |
|---|---|
| Vazyme #NA301 | 15 µL |
| Index (i5 + i7, Vazyme #TD202) | 4 µL + 4 µL |
| Splint Oligo (10 µM) | 2 µL |
| T4-Panel (10 µM) | 2 µL |
| Panel (10 µM) | 2 µL/- |
| RNase H2 (IDT #11-03-02-03) | 3 U/- |
| T4 Ligase (Vazyme #C301) | 5 µg/- |
| ATP | 0.25 mM |
| 293T gDNA | 10 ng |
| ddH₂O | To 30 µL |

**Table 18**

| Temperature | Time/min | Number of cycles |
|---|---|---|
| 37°C | 10/15/20/25/30 | 1 |
| 50°C/60°C | 10 min | 1/- |
| 95°C | 3 min | 1 |
| 95°C | 30 s | 30 |
| 62°C | 30 s | |
| 72°C | 30 s | |
| 72°C | 3 min | 1 |
| 4°C | Hold | 1 |

**Table 19**

| Different primer sets | Primer meaning | Product length (bp) |
|---|---|---|
| Panel | Third oligonucleotide primer; including a gene-specific primer sequence and a sequencing primer sequence | 367 |
| T4-Panel | Third oligonucleotide primer; including a gene-specific primer sequence and a universal sequence | 358 |
| Index | First oligonucleotide primer, including a sequencing adapter sequence, an index sequence, and a sequencing primer sequence | No amplification product |
| Index-T4-Panel | Long primer including a sequencing adapter sequence, an index sequence, a sequencing primer sequence, a universal sequence, and a gene-specific primer sequence | 456 |

Analysis of results: In this example, the amplification efficiency of the Index-T4-Panel long primer generated by ligation with T4 DNA ligase for target fragments was further tested under different conditions. As shown in FIG. 9, the amplification product of the connected Index-T4-Panel long primer (456 bp) was larger than that of the unconnected Panel primer (367 bp). Based on the brightness of the amplification product bands, it can be concluded that the amplification efficiency of the Index-T4-Panel long primer reached a level comparable to that of the Panel primer. In addition, the electrophoresis bands of test groups 1-2 and test group 3 indicated that whether RNase H2 was used to inactivate the bridge primer had no effect on the amplification results, meaning that the bridge primer did not exert any other influence on the amplification system. In summary, the method of first connecting the Index primer and the T4-Panel primer via T4 DNA ligase to form a long primer, followed by amplification, is feasible.

### Example 7

### Testing of Different Library Structures by T4 DNA Ligase-Mediated One-Step Amplification

To verify that the final library constructed by the single-tube amplicon library construction method provided by the present disclosure meets the requirements of the Illumina sequencing platform, we designed a hybrid library structure. Specifically, one end was the TruSeq library structure, and the other end was the Nextera library structure, thereby enabling normal compatibility with sequencing on the Illumina sequencer. For the 5NT-7TS group (i5 with the Nextera structure, i7 with the TruSeq structure), the bridge primers were Splint-1 and Splint-2; the first oligonucleotide primers were the Vazyme #TD202 N5xx primer and the Vazyme #N321 DM7xx primer; and the third oligonucleotide primers were T4-Panel-F1 and T4-Panel-F2. For the 5TS-7NT group (i5 with the TruSeq structure, i7 with the Nextera structure), the bridge primers were Splint-3 and Splint-4; the first oligonucleotide primers were the Vazyme #N321 DM5xx primer and the Vazyme #TD204 N9xx primer; and the third oligonucleotide primers were T4-Panel-F3 and T4-Panel-F4. The corresponding primer sequences are as follows:
Splint-1 (SEQ ID NO. 13): TCTTATACACATCTGACGCTGCCG-C3 Spacer
Splint-2 (SEQ ID NO. 14): GACAGGATGCAGATCGGAAGAGC-C3 Spacer
Splint-3 (SEQ ID NO. 15): CTAGGGACTTAGATCGGAAGAGC-C3 Spacer
Splint-4 (SEQ ID NO. 16): TCTTATACACATCTCCGAGCCCAC-C3 Spacer
T4-Panel-F1 (SEQ ID NO. 17): 5p-AGATGTGTATAAGAGACAGTCACAAATATAAATCCATTGATAAGCA
T4-Panel-F2 (SEQ ID NO. 18): 5p-GCATCCTGTCCTTTATATACCTTTGAAATATCCTAATGGG
T4-Panel-F3 (SEQ ID NO. 19): 5p-AAGTCCCTAGTCACAAATATAAATCCATTGATAAGCA
T4-Panel-F4 (SEQ ID NO. 20): 5p-
   AGATGTGTATAAGAGACAGCTTTATATACCTTTGAAATATCCTAATGGG

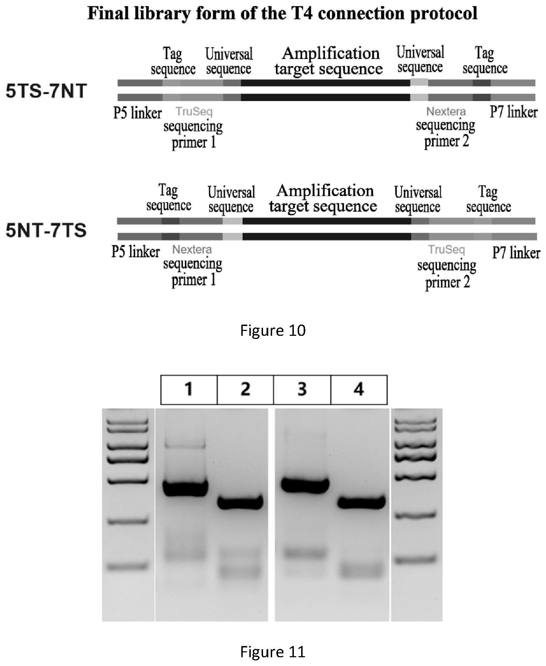

The primer addition settings for each group are shown in Table 20. The components and addition amounts of the amplification system for the 5NT-7TS group are shown in Table 21, and those for the 5TS-7NT group are shown in Table 22. The amplification reaction program is shown in Table 23. The final library structures of the 5NT-7TS and 5TS-7NT groups are shown in FIG. 10. The amplification products were subjected to agarose gel electrophoresis. The results are shown in FIG. 11.

**Table 20**

| | 5NT-7TS | | 5TS-7NT | |
|---|---|---|---|---|
| Index | Vazyme #TD202-N5xx+ | | Vazyme #N321-DM5xx+ | |
| | Vazyme #N321-DM7xx | | Vazyme #TD204-N9xx | |
| T4-Panel | T4-Panel-1, T4-Panel-2 | | T4-Panel-3, T4-Panel-4 | |
| Splint Oligo | Splint-1, Splint-2 | | Splint-3, Splint-4 | |
| T4 ligase + ATP | 5 µg + 0.25 mM | / | 5 µg + 0.25 mM | / |
| Template | 293T gDNA | | | |
| Test group | 1 | 2 | 3 | 4 |

**Table 21**

| Component | Volume |
|---|---|
| Vazyme #NA301 | 15 µL |
| Index i5, Vazyme #TD202 N5xx | 4 µL |
| Index i7, Vazyme #N321 DM7xx | 4 µL |
| Splint Oligo (10 µM) | 2 µL |
| T4-Panel (10 µM) | 2 µL |
| T4 Ligase (Vazyme #C301) | 5 µg/- |
| 293T gDNA | 10 ng |
| ATP | 0.25 mM/- |
| ddH₂O | To 30 µL |

**Table 22**

| Component | Volume |
|---|---|
| Vazyme #NA301 | 15 µL |
| Index i5, Vazyme #TD202-N7xx | 4 µL |
| Index i7, Vazyme #N321-DM5xx | 4 µL |
| Splint Oligo (10 µM) | 2 µL |
| T4-Panel (10 µM) | 2 µL |
| T4 Ligase (Vazyme #C301) | 5 µg/- |
| 293T gDNA | 10 ng |
| ATP | 0.25 mM/- |
| ddH₂O | To 30 µL |

**Table 23**

| Temperature | Time | Number of cycles |
|---|---|---|
| 37°C | 10 min | 1 |
| 95°C | 3 min | 1 |
| 95°C | 30 s | 30 |
| 62°C | 30 s | |
| 72°C | 30 s | |
| 72°C | 3 min | 1 |
| 4°C | Hold | 1 |

As shown in FIG. 11, the bridge primers designed for both the 5NT-7TS and 5TS-7NT library structures can effectively connect the corresponding T4-Panel and Index primers into Index-T4-Panel long primers, and achieve one-step efficient amplification of the complete library structures.

### Example 8

### Testing of T4 DNA Ligase-Mediated One-Step Multiplex Panel Amplification

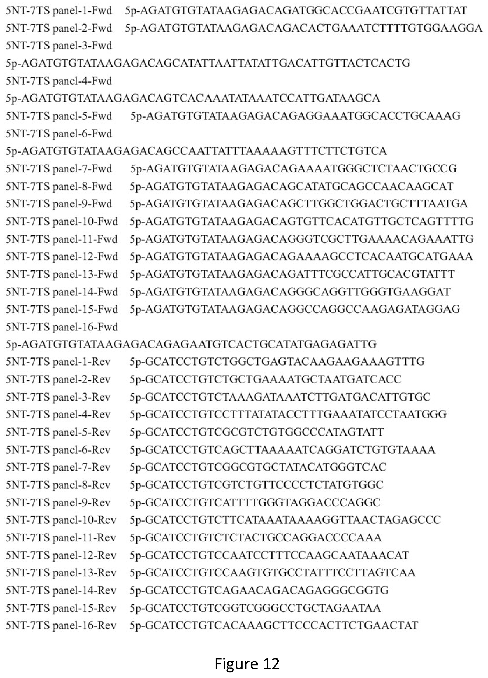

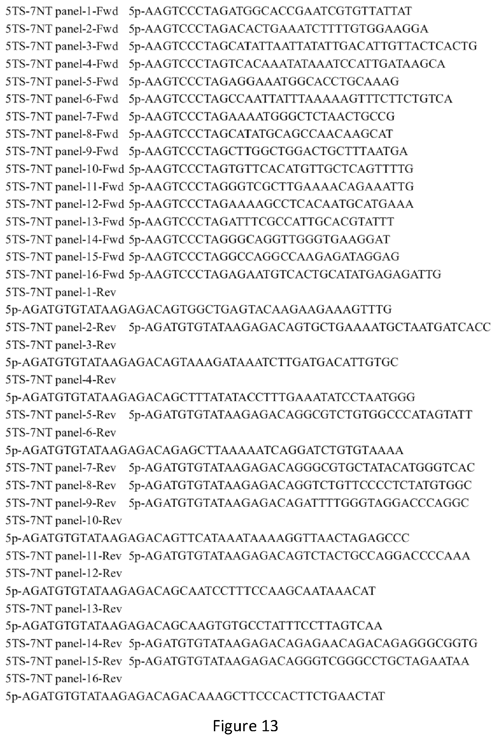

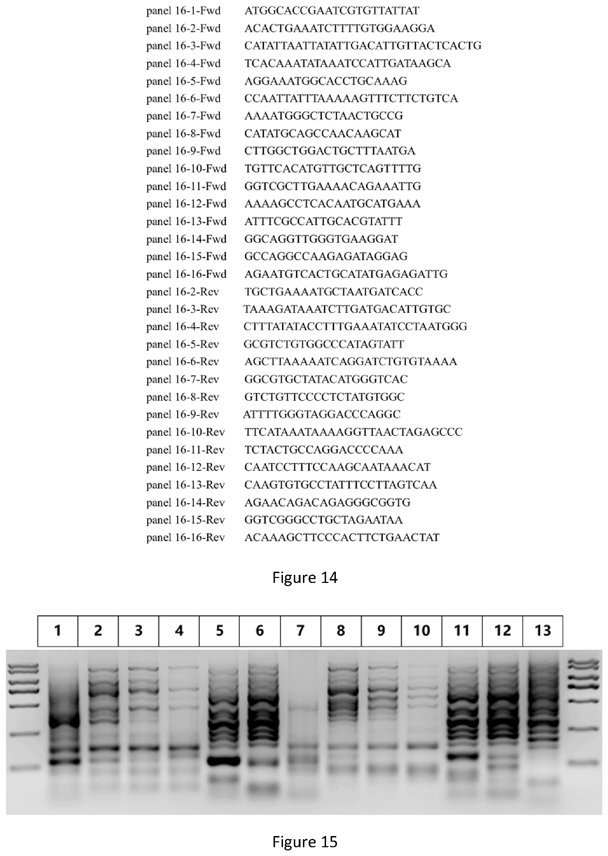

To verify the one-step, single-tube amplification strategy for constructing multiplex amplicon libraries in the present disclosure, we further designed 16-plex Panel primers for testing. The sequences of the T4-Panel-16 primers for constructing the 5NT-7TS library structure are shown in FIG. 12, the sequences of the T4-Panel-16 primers for constructing the 5TS-7NT library structure are shown in FIG. 13, and the sequences of the Panel-16 primers containing only the specific sequences are shown in FIG. 14. The sequences of the bridge primers used in the test were the same as those in Example 7. The test groups were set up according to Table 24, the one-step, single-tube amplification system was prepared according to Table 25, and the reaction was performed according to the amplification program shown in Table 26. The amplification products were subjected to gel electrophoresis to detect the amplification effect. The results are shown in FIG. 15.

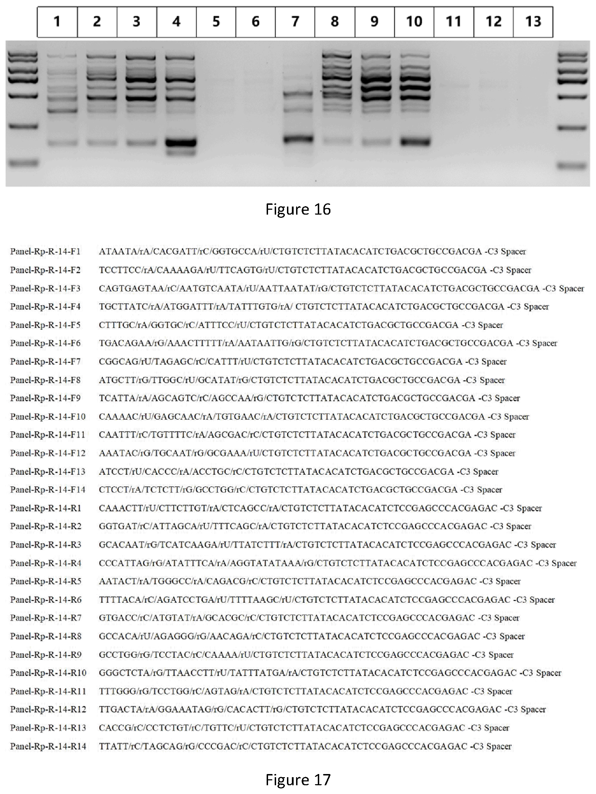

The amplification products from the above 16-plex Panel one-step amplification were recovered, and amplification was performed using the P5 and P7 sequences of the library to verify whether the one-step amplification products all contained effective libraries of the 16-plex Panel. 50 ng of the recovered products was taken, the amplification system was prepared according to Table 27, and amplification was performed according to Table 28. The amplification products were subjected to agarose gel electrophoresis to detect bands. The results are shown in FIG. 16. The sequences of the P5 primer and P7 primer are respectively as follows:
P5 (SEQ ID NO. 21): AATGATACGGCGACCACCGAGA
P7 (SEQ ID NO. 22): CAAGCAGAAGACGGCATACGAG

**Table 24**

| Index | TD202-N5xx + N321-DM7xx | | | | | | TD204-N9xx + N321-DM5xx | | | | | | / |
|---|---|---|---|---|---|---|---|---|---|---|---|---|---|
| T4-Panel-16 | 5NT-7TS T4-Panel-16 | | | | | | 5TS-7NT T4-Panel-16 | | | | | | Panel-16 |
| Index : Panel | 1: 1 | 2 : 1 | 3: 1 | 4: 1 | / | / | 1: 1 | 2 : 1 | 3: 1 | 4: 1 | / | / | / |
| Splint Oligo | Splint-1/2 | | | | | / | Splint-3/4 | | | | | / | / |
| T4 ligase + ATP | 5 µg + 0.25 mM | | | | / | / | 5 µg + 0.25 mM | | | | / | / | / |
| Test group | 1 | 2 | 3 | 4 | 5 | 6 | 7 | 8 | 9 | 10 | 11 | 12 | 13 |

**Table 25**

| Component | Addition amount (30 µL) |
|---|---|
| PCR mix (Vazyme #NA301) | 15 µL |
| T4-Panel-16 (100 µM) | 0.2 µL |
| Splint Oligo (100 µM) | 0.2 µL/- |
| Index (10 µM) | 10 µL + 10 µL/- |
| T4 ligase + ATP | 5 µg + 0.25 mM/- |
| 293T gDNA | 10 ng |
| ddH₂O | to 30 µL |

**Table 26**

| Temperature | Time | Number of cycles |
|---|---|---|
| 37°C | 10 min | 1 |
| 95°C | 3 min | 1 |
| 95°C | 30 s | 30 |
| 62°C | 30 s | |
| 72°C | 1 min | |
| 72°C | 3 min | 1 |

**Table 27**

| Amplification After Product Recovery | |
|---|---|
| Component | Addition amount (40 µL) |
| PCR mix (Vazyme #N616) | 20 µL |
| P5 + P7 (10 µM) | 2 µL |
| Recovered product | 50 ng |
| ddH₂O | to 40 µL |

**Table 28**

| Temperature | Time | Number of cycles |
|---|---|---|
| 95°C | 3 min | 1 |
| 95°C | 30 s | 6 |
| 60°C | 30 s | |
| 72°C | 1 min | |
| 72°C | 3 min | 1 |

As shown in FIG. 15, for both the 5NT-7TS and 5TS-7NT library structure types, the 16-plex T4-Panel primers were able to achieve the extension of long primers and the amplification of target fragments. Moreover, the electrophoresis bands of the amplification products increased in intensity with increasing amounts of Index primer used. However, a further increase in the Index primer inhibited the amplification of the multiplex Panel targets.

As shown in FIG. 16, further amplification was performed on the one-step amplification products using P5 and P7 primers. The results showed that the amplification products from the one-step amplification test groups were all able to be amplified by P5 and P7 primers (test groups 1-4 and 7-10), whereas products from non-one-step amplification systems were not able to be amplified by P5 and P7 primers (test groups 5, 6, 11, 12, and 13). Moreover, as the amount of Index primer in the system increased, the yield of the P5 and P7 amplification products also increased. However, a further increase in the Index primer inhibited the amplification of the target product. This indicates that in the T4 DNA ligase-mediated one-step amplification system, the molar ratio of Index primer to T4-Panel primer can range from 1 : 1 to 4 : 1, with the optimal ratio being from 2 : 1 to 3 : 1.

### Example 9

### One-Step Multiplex Panel Amplification Mediated by Reverse Complementary Panel Primers With RNA Modifications

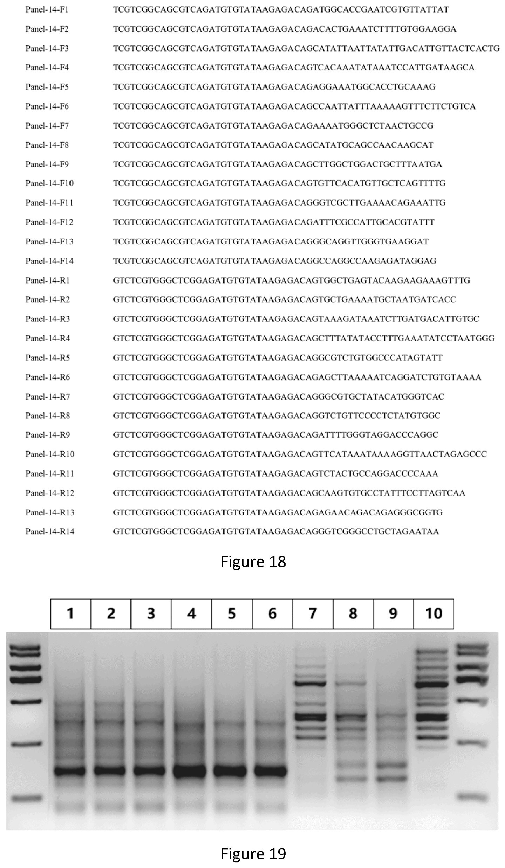

To verify the one-step amplification strategy mediated by reverse complementary primers with RNA modifications for constructing multiplex amplicon libraries in the present disclosure, we further designed 14-plex reverse Panel primers with RNA modifications (Panel-Rp-R-14) for testing. The sequences of the 14-plex reverse Panel primers with RNA modifications (Panel-Rp-R-14) and the sequences of the normal Panel primers are shown in FIG. 17 and FIG. 18, respectively. The test groups were set up according to Table 29, the one-step, single-tube amplification system was prepared according to Table 30, and the reaction was performed according to the amplification program shown in Table 31. The amplification products were subjected to gel electrophoresis to detect the amplification effect. The results are shown in FIG. 19.

**Table 29**

| | Index + Panel-Rp-R-14 | | | Index + Panel-Rp-R-14 | | | Index + Panel | | | Panel |
|---|---|---|---|---|---|---|---|---|---|---|
| DNA Pol | Phanta DNA polymerase | | | | | | | | | |
| RNase H2 | 3 U | | | 3 U | | | / | | | / |
| Index : Panel (Panel-RP) | 1:1 | | | 2:1 | | | 1:1 | 2 : 1 | 4: 1 | / |
| Treatment time | 37°C 10 min + 72°C 10 min | | | | | | | | | |
| Test group | 1 | 2 | 3 | 4 | 5 | 6 | 7 | 8 | 9 | 10 |

**Table 30**

| Component | Volume |
|---|---|
| Vazyme #NA301 | 15 µL |
| Index, i5 + i7 (Vazyme #TD202) | 2.5 µL/5 µL/10 µL/- |
| Panel-Rp-R-14 (20 µM) | 1 µL/- |
| Panel (20 µM) | 1 µL/- |
| Phanta DNA polymerase(Vazyme #P501) | 1 U |
| RNase H2 (IDT #11-03-02-03) | 3 U/- |
| 293T gDNA | 10 ng |
| ddH₂O | To 30 µL |

**Table 31**

| Temperature | Time | Number of cycles |
|---|---|---|
| 37°C | 10 min | 1 |
| 72°C | 10 min | 1 |
| 95°C | 3 min | 1 |
| 95°C | 30 s | 30 |
| 62°C | 30 s | |
| 72°C | 30 s | |
| 72°C | 3 min | 1 |
| 4°C | Hold | 1 |

As shown in FIG. 19, when the molar ratio of the Index primer to the reverse Panel primer with RNA modifications was 1 : 1, the one-step, single-tube multiplex amplification mediated by the reverse Panel primer with RNA modifications achieved the best effect. In contrast, direct amplification using a mixture of conventional Panel primers and Index primers yielded fewer target-specific products, resulting in a lower proportion of effective libraries. In summary, the test results of the 14-plex reverse Panel with RNA modifications indicate that the one-step, single-tube method mediated by reverse Panel primers with RNA modifications is suitable for multiplex amplification.

### Example 10

### One-Step Multiplex Panel Amplification Mediated by Reverse Complementary Panel Primers With RNA Modifications

To verify the one-step amplification strategy mediated by reverse complementary primers with RNA modifications for constructing multiplex amplicon libraries in the present disclosure, we further designed 223-plex reverse Panel primers with RNA modifications (Panel-Rp-R-223) for testing. Among them, 100-plex primers targeted the genome of *Escherichia coli*, 100-plex primers targeted *Staphylococcus aureus*, and the remaining 23-plex primers targeted the genome of *Komagataella pastoris*. The sequences of the 223-plex reverse Panel primers with RNA modifications are respectively as follows:
SEQ ID NO. 27 (Panel-Rp-223-F1):
   CAGCCA/rU/TTGAC/rA/CGTCTC/rG/CTGTCTCTTATACACATCTGACGCTGCCGACGA
SEQ ID NO. 28 (Panel-Rp-223-F2):
   TACCGA/rU/TTGCG/rU/CGTACC/rA/CTGTCTCTTATACACATCTGACGCTGCCGACGA
A total of 223 pairs are included, with two upstream primers listed here as examples.

The sequences of 223-plex normal Panel primers are respectively as follows:
SEQ ID NO. 25 (Panel-TD-223-F1):
   TCGTCGGCAGCGTCAGATGTGTATAAGAGACAGCGAGACGTGTCAAATGGCTG
SEQ ID NO. 26 (Panel-TD-223-F2):
   TCGTCGGCAGCGTCAGATGTGTATAAGAGACAGTGGTACGACGCAAATCGGTA

A total of 223 pairs are included, all sharing the same sequencing primer sequence at the 5' end, with different gene-specific sequences designed based on the target sites (two upstream sequences are listed here as examples).

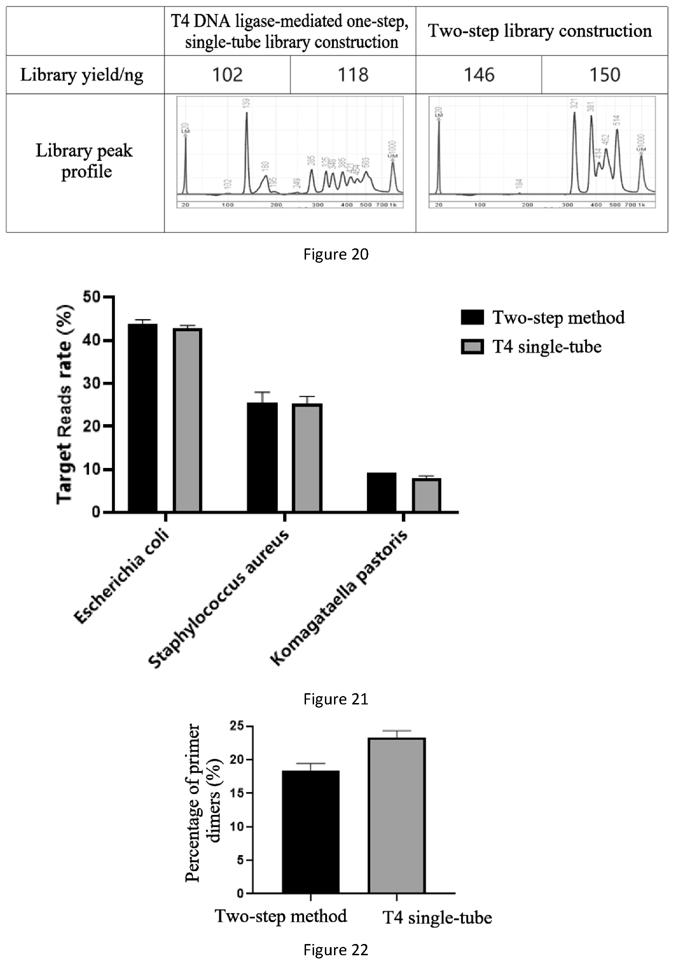

The pathogen template was prepared as pathogen-simulated sample DNA by mixing 293T gDNA with genomic DNA of *Escherichia coli*, genomic DNA of *Staphylococcus aureus*, and genomic DNA of *Komagataella pastoris* at a ratio of 1000 : 1 (1 µg of 293T gDNA + 1 ng of *Escherichia coli*, 1 ng of *Staphylococcus aureus*, and 1 ng of *Komagataella pastoris*). The systems for the two-step method are shown in Tables 38 and 39, and the single-tube system containing reverse Panel primers with RNA modifications is shown in Table 40. The amplification programs for the two-step method are shown in Tables 42 and 43, and the single-tube amplification program is shown in Table 41. The amplified libraries were subjected to sequencing on the platform. The number of reads detected from sequencing analysis is shown in FIG. 20, and the number of dimers is shown in FIG. 21.

**Table 38**

| First-round amplification system for the two-step method | |
|---|---|
| Component | Volume |
| Vazyme #NA301 | 15 µL |
| Panel-TD-223 (100 µM/446) | 2 µL |
| Pathogen-simulated sample DNA (1 ng) | 1 µL |
| ddH₂O | To 30 µL |

**Table 39**

| Second-round amplification system for the two-step method | |
|---|---|
| Component | Volume |
| First-round amplification product | 20 ng |
| Vazyme #N618 | 20 µL |
| Index i5 + i7 (Vazyme #TD202) | 2 + 2 µL |
| ddH₂O | To 40 µL |

**Table 40**

| Component | Volume |
|---|---|
| Vazyme #NA301 | 15 µL |
| Index, i5 + i7 (Vazyme #TD202) | 10 µL |
| Panel-Rp-R-223 (50 µM) | 3 µL |
| Phanta DNA polymerase(Vazyme #P501) | 1 U |
| RNase H2 (IDT #11-03-02-03) | 3 U |
| Pathogen-simulated sample DNA (1 ng) | 1 µL |
| ddH₂O | To 50 µL |

**Table 41**

| One-step, single-tube amplification program | | |
|---|---|---|
| Temperature | Time | Number of cycles |
| 37°C | 10 min | 1 |
| 95°C | 3 min | 1 |
| 95°C | 30 s | 30 |
| 60°C | 30 s | |
| 72°C | 30 s | |
| 72°C | 3 min | 1 |
| 4°C | Hold | 1 |

**Table 42**

| First-round amplification program for the two-step method | | |
|---|---|---|
| Temperature | Time | Number of cycles |
| 95°C | 3 min | 1 |
| 95°C | 30 s | 25 |
| 60°C | 30 s | |
| 72°C | 1 min | |
| 72°C | 3 min | 1 |
| 4°C | Hold | 1 |

**Table 43**

| Second-round amplification program for the two-step method | | |
|---|---|---|
| Temperature | Time | Number of cycles |
| 95°C | 3 min | 1 |
| 95°C | 30 s | 5 |
| 60°C | 30 s | |
| 72°C | 1 min | |
| 72°C | 3 min | 1 |
| 4°C | Hold | 1 |

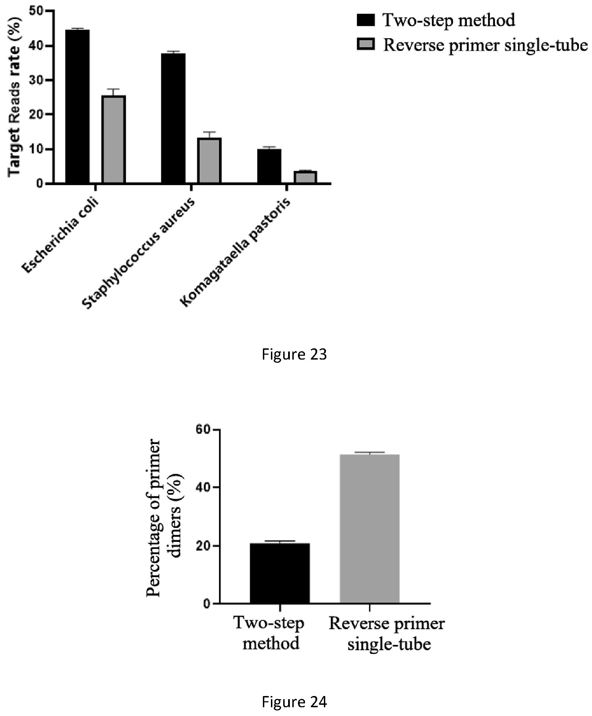

As shown in FIGs. 23 and 24, the single-tube library construction strategy mediated by the 223-plex reverse Panel primers with RNA modifications was also able to detect target reads. The number of detected species-target reads was slightly lower than that of the two-step method, and the number of primer dimers was slightly higher than that of the two-step method. However, the targets were still effectively amplified. This indicates that the multiplexed amplicon library construction method mediated by reverse complementary Panel primers with RNA modifications can effectively amplify target fragments for library construction.

### Example 11

### Testing of T4 DNA Ligase-Mediated One-Step 223-Plex Panel Amplification

To verify the ability of the T4 ligase-mediated one-step amplification method for constructing multiplex amplicon libraries in the present disclosure to construct multiplex amplicon libraries under ultra-multiplex conditions, we further designed 223-plex T4-Panel primers (T4-Panel-223) with the 5TS-7NT library structure. Among them, 100-plex primers targeted the genome of *Escherichia coli*, 100-plex primers targeted *Staphylococcus aureus*, and the remaining 23-plex primers targeted the genome of *Komagataella pastoris*. The sequences of the 223-plex Panel primers with the 5TS-7NT library structure are as follows:
SEQ ID NO. 23 (T4-Panel-223-F1):
   5P-GCATCCTGTCCGAGACGTGTCAAATGGCTG
SEQ ID NO. 24 (T4-Panel-223-F2):
   5P-GCATCCTGTCTGGTACGACGCAAATCGGTA

A total of 223 pairs are included, all sharing the same universal sequence at the 5' end, with different gene-specific sequences designed based on the target sites (two upstream sequences are listed here as examples).

The sequences of 223-plex normal Panel primers are respectively as follows:
SEQ ID NO. 25 (TD-Panel-223-F1):
   TCGTCGGCAGCGTCAGATGTGTATAAGAGACAGCGAGACGTGTCAAATGGCTG
SEQ ID NO. 26 (TD-Panel-223-F2):
   TCGTCGGCAGCGTCAGATGTGTATAAGAGACAGTGGTACGACGCAAATCGGTA

A total of 223 pairs are included, all sharing the same sequencing primer sequence at the 5' end, with different gene-specific sequences designed based on the target sites (two upstream sequences are listed here as examples).

The sequences of the bridge primers Splint 3 and Splint 4 are as shown in Example 7. The pathogen template was prepared as pathogen-simulated sample DNA by mixing 293T gDNA with genomic DNA of *Escherichia coli*, genomic DNA of *Staphylococcus aureus*, and genomic DNA of *Komagataella pastoris* at a ratio as follows: 10 µg of 293T + 1 ng of *Escherichia coli*, 1 ng of *Staphylococcus aureus*, and 1 ng of *Komagataella pastoris* gDNA. The amplification system was prepared according to Table 32, and amplification was performed according to the amplification program shown in Table 35. The amplification system for the conventional two-step method was prepared according to Tables 33 and 34, and the reaction programs were performed according to Tables 36 and 37, respectively. The amplification products were recovered. The concentrations of the products were determined using Qubit, and the peak profiles of the products were analyzed using Qseq, followed by sequencing on the platform. The Qseq peak profiles of the libraries are shown in FIG. 20, the number of reads detected from sequencing analysis is shown in FIG. 21, and the number of dimers is shown in FIG. 22.

**Table 32**

| One-step, single-tube method | |
|---|---|
| Component | Volume |
| Vazyme #NA301 | 15 µL |
| T4-Panel-223 (100 µM/446) | 2 µL |
| Index i5 + i7 (Vazyme #N321-DM5xx+ Vazyme #TD204-N9xx) | 2 µL+ 2 µL |
| Splint Oligo 3 + 4 (100 µM + 100 µM) | 2 µL |
| T4 Ligase (Vazyme #C301) (5 µg) | 1 µL |
| Pathogen-simulated sample DNA (1 ng) | 1 µL |
| ATP (0.25 mM) | 1 µL |
| ddH₂O | To 30 µL |

**Table 33**

| First-round amplification system for the two-step method | |
|---|---|
| Component | Volume |
| Vazyme #NA301 | 15 µL |
| TD-Panel-223 (100 µM/446) | 2 µL |
| Pathogen-simulated sample DNA (1 ng) | 1 µL |
| ddH₂O | To 30 µL |

**Table 34**

| Second-round amplification system for the two-step method | |
|---|---|
| Component | Volume |
| First-round amplification product | 20 ng |
| Vazyme #N618 | 20 µL |
| Index i5 + i7 (Vazyme #TD202) | 2 + 2 µL |
| ddH₂O | To 40 µL |

**Table 35**

| One-step, single-tube amplification program | | |
|---|---|---|
| Temperature | Time | Number of cycles |
| 37°C | 10 min | 1 |
| 95°C | 3 min | 1 |
| 95°C | 30 s | 30 |
| 60°C | 30 s | |
| 72°C | 30 s | |
| 72°C | 3 min | 1 |
| 4°C | Hold | 1 |

**Table 36**

| First-round amplification program for the two-step method | | |
|---|---|---|
| Temperature | Time | Number of cycles |
| 95°C | 3 min | 1 |
| 95°C | 30 s | 25 |
| 60°C | 30 s | |
| 72°C | 1 min | |
| 72°C | 3 min | 1 |
| 4°C | Hold | 1 |

**Table 37**

| Second-round amplification program for the two-step method | | |
|---|---|---|
| Temperature | Time | Number of cycles |
| 95°C | 3 min | 1 |
| 95°C | 30 s | 5 |
| 60°C | 30 s | |
| 72°C | 1 min | |
| 72°C | 3 min | 1 |
| 4°C | Hold | 1 |

As shown in FIG. 20, the target product peak profiles of the T4 DNA ligase-mediated single-tube targeted multiplexed amplicon library were clearly resolved. Further, high-throughput sequencing data from FIGs. 20 and 21 showed that the number of target reads detected for the three pathogens was consistent between the single-tube method and the two-step method. The number of primer dimers in the T4 DNA ligase-mediated one-step, single-tube library construction was slightly higher than that in the two-step method.

## Claims

1. An oligonucleotide set, comprising a first oligonucleotide primer and a second oligonucleotide primer, wherein the first oligonucleotide primer comprises, sequentially from the 5' end to the 3' end, a sequencing adapter sequence, an index sequence, and a sequencing primer sequence, and the second oligonucleotide primer comprises, sequentially from the 5' end to the 3' end, a reverse complementary sequence of a gene-specific primer, and a reverse complementary sequence of the sequencing primer.

2. The oligonucleotide set according to claim 1, wherein one or more nucleotide sites of the reverse complementary sequence of the gene-specific primer contain an RNA base modification.

3. The oligonucleotide set according to claim 1, wherein the 3' end of the second oligonucleotide primer contains a blocking modification, preferably a 3' phosphorylation modification or a spacer arm modification, and more preferably a Spacer C3 modification, a Spacer C6 modification, a Spacer C9 modification, a dSpacer modification, or a PC-linker modification.

4. The oligonucleotide set according to claim 2, wherein the first oligonucleotide primer comprises an upstream first oligonucleotide primer and a downstream first oligonucleotide primer, the upstream first oligonucleotide primer comprising, sequentially from the 5' end to the 3' end, an upstream sequencing adapter sequence, an upstream index sequence, and an upstream sequencing primer sequence, and the downstream first oligonucleotide primer comprising, sequentially from the 5' end to the 3' end, a downstream sequencing adapter sequence, a downstream index sequence, and a downstream sequencing primer sequence; the second oligonucleotide primer comprises an upstream second oligonucleotide primer and a downstream second oligonucleotide primer, the upstream second oligonucleotide primer comprising, sequentially from the 5' end to the 3' end, a reverse complementary sequence of a gene-specific upstream primer and a reverse complementary sequence of the upstream sequencing primer, and the downstream second oligonucleotide primer comprising, sequentially from the 5' end to the 3' end, a reverse complementary sequence of a gene-specific downstream primer and a reverse complementary sequence of the downstream sequencing primer; preferably, the upstream sequencing adapter sequence is a P5 adapter sequence of the Illumina platform, and the downstream sequencing adapter sequence is a P7 adapter sequence of the Illumina platform.

5. The oligonucleotide set according to claim 4, wherein the first oligonucleotide primer comprises at least one pair of an upstream first oligonucleotide primer and a downstream first oligonucleotide primer, and the second oligonucleotide primer comprises at least one pair of an upstream second oligonucleotide primer and a downstream second oligonucleotide primer.

6. A composition, comprising a DNA polymerase, an endoribonuclease, dNTPs, a buffer component, a metal salt, and a primer combination, wherein the primer combination comprises the oligonucleotide set according to claim 5.

7. The composition according to claim 6, wherein the composition further comprises a DNA sample; preferably, the DNA sample is gDNA or cDNA.

8. The composition according to claim 6, wherein the DNA polymerase comprises at least one thermostable DNA polymerase; preferably, the DNA polymerase comprises at least one thermostable DNA polymerase and one mesophilic DNA polymerase.

9. The composition according to claim 6, wherein the endoribonuclease is an endonuclease capable of recognizing a ribonucleotide site and cleaving a phosphodiester bond; preferably, the endoribonuclease is RNase H2 or a homologous protein of RNase H2.

10. A multiplexed amplicon library construction method, comprising steps of:
(1) preparing a reaction system comprising at least one pair of first oligonucleotide primers, at least one pair of second oligonucleotide primers, and a sample nucleic acid, wherein the first oligonucleotide primer comprises, sequentially from the 5' end to the 3' end, a sequencing adapter sequence, an index sequence, and a sequencing primer sequence, the second oligonucleotide primer comprises, sequentially from the 5' end to the 3' end, a reverse complementary sequence of a gene-specific primer and a reverse complementary sequence of the sequencing primer, and the second oligonucleotide primer comprises a cleavable group;
(2) subjecting the 3' end of the first oligonucleotide primer and the 3' end of the second oligonucleotide primer to reverse complementary pairing, and extending the first oligonucleotide primer in the 5'-3' direction to obtain an extension product;
(3) cleaving the reverse complementary sequence of the gene-specific primer in the second oligonucleotide primer; and
(4) performing PCR amplification on the sample nucleic acid using the extension product described in step (2) as a primer.

11. The method according to claim 10, wherein the 3' end of the second oligonucleotide primer contains a blocking modification, preferably a 3' phosphorylation modification or a spacer arm modification, and more preferably a Spacer C3 modification, a Spacer C6 modification, a Spacer C9 modification, a dSpacer modification, or a PC-linker modification.

12. The method according to claim 10, wherein the at least one pair of first oligonucleotide primers comprises at least one pair of an upstream first oligonucleotide primer and a downstream first oligonucleotide primer, the upstream first oligonucleotide primer comprising, sequentially from the 5' end to the 3' end, an upstream sequencing adapter sequence, an upstream index sequence, and an upstream sequencing primer sequence, and the downstream first oligonucleotide primer comprising, sequentially from the 5' end to the 3' end, a downstream sequencing adapter sequence, a downstream index sequence, and a downstream sequencing primer sequence; the at least one pair of second oligonucleotide primers comprises at least one pair of an upstream second oligonucleotide primer and a downstream second oligonucleotide primer, the upstream second oligonucleotide primer comprising, sequentially from the 5' end to the 3' end, a reverse complementary sequence of a gene-specific upstream primer and a reverse complementary sequence of the upstream sequencing primer, and the downstream second oligonucleotide primer comprising, sequentially from the 5' end to the 3' end, a reverse complementary sequence of a gene-specific downstream primer and a reverse complementary sequence of the downstream sequencing primer; preferably, the upstream sequencing adapter sequence is a P5 adapter sequence of the Illumina platform, and the downstream sequencing adapter sequence is a P7 adapter sequence of the Illumina platform.

13. The method according to claim 10, wherein the sample nucleic acid is DNA; preferably, the DNA is gDNA or cDNA.

14. The method according to claim 10, wherein the cleavable group is an RNA base, and the RNA base is at least one of rA (riboadenosine), rU (ribouridine), rG (riboguanosine), and rC (ribocytidine).

15. The method according to claim 14, wherein the reaction system further comprises an endoribonuclease, wherein the endoribonuclease is an endonuclease capable of recognizing a ribonucleotide site and cleaving a phosphodiester bond; preferably, the endoribonuclease is RNase H2 or a homologous protein of RNase H2.

16. The method according to claim 15, wherein the reaction system further comprises a DNA polymerase; preferably, the DNA polymerase comprises at least one thermostable DNA polymerase.

17. The method according to claim 16, wherein the DNA polymerase comprises at least one thermostable DNA polymerase and one mesophilic DNA polymerase.

18. The method according to claim 16, wherein the DNA polymerase comprises at least one non-hot-start polymerase and one hot-start polymerase.

19. The method according to claim 17, wherein the reaction of step (2) is performed under the action of a mesophilic polymerase; and the reaction of step (4) is performed under the action of a thermostable polymerase.

20. The method according to claim 18, wherein the reaction of step (2) is performed under the action of a non-hot-start polymerase; and the reaction of step (4) is performed under the action of a hot-start polymerase.

21. The method according to any one of claims 19 and 20, wherein conditions for the reaction of step (2) are incubation at 25-40°C for 10-30 min, and the PCR amplification of step (4) comprises denaturation, annealing, and extension.

22. The method according to claim 15, wherein conditions for the reaction of step (3) are incubation at 60-80°C for 10-20 min.

23. The method according to claim 10, wherein the reaction system further comprises one or more of a metal salt, a buffer component, and dNTPs.

24. An oligonucleotide primer, comprising, sequentially from the 5' end to the 3' end, a sequencing adapter sequence, an index sequence, a sequencing primer sequence, and a gene-specific primer sequence.

25. An oligonucleotide combination, comprising a first oligonucleotide primer, a third oligonucleotide primer, and a bridge primer, wherein the first oligonucleotide primer comprises, sequentially from the 5' end to the 3' end, a sequencing adapter sequence, an index sequence, and a sequencing primer sequence, the third oligonucleotide primer comprises, sequentially from the 5' end to the 3' end, a universal sequence and a gene-specific primer sequence, and the bridge primer comprises, sequentially from the 5' end to the 3' end, a reverse complementary sequence of the universal sequence and a reverse complementary sequence of the sequencing primer.

26. The oligonucleotide combination according to claim 25, wherein the 3' end of the bridge primer contains a blocking modification, preferably a 3' phosphorylation modification or a spacer arm modification, and more preferably a Spacer C3 modification, a Spacer C6 modification, a Spacer C9 modification, a dSpacer modification, or a PC-linker modification.

27. The oligonucleotide combination according to claim 26, wherein the first oligonucleotide primer comprises an upstream first oligonucleotide primer and a downstream first oligonucleotide primer, the upstream first oligonucleotide primer comprising, sequentially from the 5' end to the 3' end, an upstream sequencing adapter sequence, an upstream index sequence, and an upstream sequencing primer sequence, and the downstream first oligonucleotide primer comprising, sequentially from the 5' end to the 3' end, a downstream sequencing adapter sequence, a downstream index sequence, and a downstream sequencing primer sequence; the third oligonucleotide primer comprises an upstream third oligonucleotide primer and a downstream third oligonucleotide primer, the upstream third oligonucleotide primer comprising, sequentially from the 5' end to the 3' end, an upstream universal sequence and a gene-specific upstream primer sequence, and the downstream third oligonucleotide primer comprising, sequentially from the 5' end to the 3' end, a downstream universal sequence and a gene-specific downstream primer sequence; the bridge primer comprises an upstream bridge primer and a downstream bridge primer, the upstream bridge primer comprising, sequentially from the 5' end to the 3' end, a reverse complementary sequence of the upstream universal sequence and a reverse complementary sequence of the upstream sequencing primer, and the downstream bridge primer comprising, sequentially from the 5' end to the 3' end, a reverse complementary sequence of the downstream universal sequence and a reverse complementary sequence of the downstream sequencing primer.

28. The oligonucleotide combination according to claim 27, wherein the first oligonucleotide primer comprises at least one pair of an upstream first oligonucleotide primer and a downstream first oligonucleotide primer, the third oligonucleotide primer comprises at least one pair of an upstream third oligonucleotide primer and a downstream third oligonucleotide primer, and the bridge primer comprises at least one pair of an upstream bridge primer and a downstream bridge primer.

29. A composition, comprising a DNA polymerase, T4 DNA ligase, ATP, dNTPs, a buffer component, a metal salt, and a primer combination, wherein the primer combination comprises the oligonucleotide combination according to claim 28.

30. The composition according to claim 29, wherein the composition further comprises a DNA sample; preferably, the DNA sample is gDNA or cDNA.

31. The composition according to claim 29, wherein the 5' end of the third oligonucleotide primer contains a phosphorylation modification, or the 5' end of the third oligonucleotide primer does not contain a phosphorylation modification, and the composition further comprises T4 polynucleotide kinase.

32. The composition according to claim 29, wherein a mass ratio or molar ratio of the first oligonucleotide primer to the third oligonucleotide primer is N : 1, wherein N is a constant greater than or equal to 1 and less than or equal to 5.

33. A multiplexed amplicon library construction method, comprising steps of:
(1) preparing a reaction system comprising at least one pair of first oligonucleotide primers, at least one pair of third oligonucleotide primers, at least one pair of bridge primers, and a sample nucleic acid, wherein the first oligonucleotide primer comprises, sequentially from the 5' end to the 3' end, a sequencing adapter sequence, an index sequence, and a sequencing primer sequence, the third oligonucleotide primer comprises, sequentially from the 5' end to the 3' end, a universal sequence and a gene-specific primer sequence, and the bridge primer comprises, sequentially from the 5' end to the 3' end, a reverse complementary sequence of the universal sequence and a reverse complementary sequence of the sequencing primer;
(2) subjecting the 3' end of the first oligonucleotide primer and the 3' end of the bridge primer to reverse complementary pairing, subjecting the 5' end of the third oligonucleotide primer and the 5' end of the bridge primer to reverse complementary pairing, and connecting the first oligonucleotide primer and the third oligonucleotide primer to obtain a ligation product; and
(3) performing PCR amplification on the sample nucleic acid using the ligation product described in step (2) as a primer.

34. The method according to claim 33, wherein the 3' end of the bridge primer contains a blocking modification, preferably a 3' phosphorylation modification or a spacer arm modification, and more preferably a Spacer C3 modification, a Spacer C6 modification, a Spacer C9 modification, a dSpacer modification, or a PC-linker modification.

35. The method according to claim 33, wherein the sample nucleic acid is DNA, preferably gDNA or cDNA.

36. The method according to claim 33, wherein the 5' end of the third oligonucleotide primer contains a phosphorylation modification, or the 5' end of the third oligonucleotide primer does not contain a phosphorylation modification, and the reaction system further comprises T4 polynucleotide kinase.

37. The method according to claim 33, wherein a mass ratio or molar ratio of the first oligonucleotide primer to the third oligonucleotide primer is N : 1, wherein N is a constant greater than or equal to 1 and less than or equal to 5.

38. The method according to claim 36, wherein the at least one pair of first oligonucleotide primers comprises at least one pair of an upstream first oligonucleotide primer and a downstream first oligonucleotide primer, the upstream first oligonucleotide primer comprising, sequentially from the 5' end to the 3' end, an upstream sequencing adapter sequence, an upstream index sequence, and an upstream sequencing primer sequence, and the downstream first oligonucleotide primer comprising, sequentially from the 5' end to the 3' end, a downstream sequencing adapter sequence, a downstream index sequence, and a downstream sequencing primer sequence; the at least one pair of third oligonucleotide primers comprises at least one pair of an upstream third oligonucleotide primer and a downstream third oligonucleotide primer, the upstream third oligonucleotide primer comprising, sequentially from the 5' end to the 3' end, an upstream universal sequence and a gene-specific upstream primer sequence, and the downstream third oligonucleotide primer comprising, sequentially from the 5' end to the 3' end, a downstream universal sequence and a gene-specific downstream primer sequence; the at least one pair of bridge primers comprises at least one pair of an upstream bridge primer and a downstream bridge primer, the upstream bridge primer comprising, sequentially from the 5' end to the 3' end, a reverse complementary sequence of the upstream universal sequence and a reverse complementary sequence of the upstream sequencing primer, and the downstream bridge primer comprising, sequentially from the 5' end to the 3' end, a reverse complementary sequence of the downstream universal sequence and a reverse complementary sequence of the downstream sequencing primer.

39. The method according to claim 33, wherein conditions for the reaction of step (2) are incubation at 20-50°C for 1-60 min.

40. The method according to claim 38, wherein the reaction system further comprises a DNA ligase, preferably T4 DNA ligase.

41. The method according to claim 40, wherein the reaction system further comprises a DNA polymerase, preferably a thermostable DNA polymerase, and more preferably Taq DNA polymerase.

42. The method according to claim 33, wherein the reaction system further comprises one or more of a metal salt, a buffer component, and dNTPs.

43. An oligonucleotide primer, comprising, sequentially from the 5' end to the 3' end, an adapter sequence, an index sequence, a sequencing primer sequence, a universal sequence, and a gene-specific primer sequence.

44. Use of the method according to any one of claims 10-23 and claims 33-42 in multiplexed amplicon library construction.
